# EUROPEAN PATENT APPLICATION

(11) **EP 3 901 160 A1**
(43) Date of publication of application: **27.10.2021**
(21) Application number: 20171442.5
(22) Date of filing: 25.04.2020
(51) Int. Cl.: C07H 19/048, C07H 21/00, A61K 31/706, A61K 31/7084, A61P 31/12, A61P 11/00

(54) **NICOTINAMIDE MONONUCLEOTIDE AND NICOTINAMIDE RIBOSIDE DERIVATIVES AND USE THEREOF IN THE TREATMENT OF VIRAL INFECTIONS AND RESPIRATORY COMPLICATIONS, IN PARTICULAR CAUSED BY INFLUENZAVIRUS OR CORONAVIRUS**

(71) Applicant: Nuvamid SA, 1003 Lausanne (CH)
(72) Inventor: BERMOND, Guillaume, 13007 Marseille (FR); GARCON, Laurent, 13960 Sausset les Pins (FR)
(74) Representative: August Debouzy

(57) **Abstract**

The present invention relates to Nicotinamide mononucleotide derivatives of formula (I) 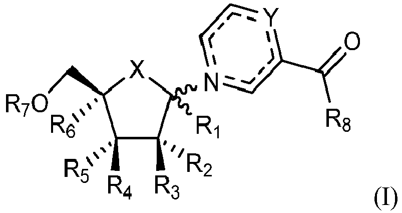 for use in the treatment and/or prevention of viral infections, such as respiratory infections The present invention further relates to pharmaceutical compositions comprising compounds of formula (I) for use in the treatment and/or prevention of viral infections.

## Description

### FIELD OF INVENTION

The present invention relates to Nicotinamide mononucleotide derivatives compounds for use in the treatment and/or prevention of viral infections.

### BACKGROUND OF INVENTION

The defense against diseases is critical for the survival of all animals, and the mechanism employed for this purpose is the animal immune system. With two main divisions involved being (i) innate immunity and (ii) adaptive immunity, the immune system is very complex. The innate immune system includes the cells and mechanisms that defend the host from infection by invading organisms, in a non-specific manner. Leukocytes, which are involved with the innate system, include *inter alia* phagocytic cells, such as macrophages, neutrophils and dendritic cells. The innate system is fully functional before a pathogen enters the host.

In contrast, the adaptive system is only initiated after the pathogen has entered the host cells, at which point it develops a pathogen-specific defense. The main cell types of the adaptive immune system are called lymphocytes, the two main categories of which are B cells and T Cells. B cells are involved in the creation of neutralizing antibodies that circulate in blood plasma and lymph and form part of the humoral immune response. T cells play a role in both the humoral immune response and the cell-mediated immunity. There are several subsets of activator or effector T cells, including cytotoxic T cells (CD8+) and "helper" T cells (CD4+), of which there are two main types known as Type 1 helper T cells (Th1) and Type 2 helper T cell (Th2).

Th1 cells promote a cell-mediated adaptive immune response, which involves the activation of macrophages and stimulates the release of various cytokines, such as IFNγ, TNF-α and IL-12, in response to an antigen. These cytokines influence the function of other cells in the adaptive and innate immune responses, and result in the destruction of micro-organisms. Generally, Th1 responses are more effective against intracellular pathogens, such as viruses and bacteria present inside host cells, while a Th2 responses are more effective against extracellular pathogens, such as parasites and toxins located outside host cells.

Amongst viral infections, acute viral infections are the most difficult to control when vaccines are not available. Antiviral therapy is often not effective if not given early in infection, as seen with influenza, measle or the frequent outbreak of norovirus gastroenteritis, which affect millions of people each year.

Amongst acute viral infections, respiratory infection is the most common type in people; causative agents including rhinoviruses, respiratory syncytial virus, influenza virus, parainfluenza virus, human metapneumovirus, measles, mumps, adenovirus and coronaviruses.

Most respiratory infections, especially those of the upper respiratory tract, are mild and not incapacitating. Upper respiratory tract infections often cause rhinorrhea or pharyngitis. Lower respiratory tract infections can be more severe and are more likely than upper respiratory tract infections to cause fever, dyspnea, chest pain or pneumonia. Cough is often present in either upper or lower respiratory tract infections.

Typical influenza in adults is characterized by sudden onset of chills, fever, prostration, cough, and generalized aches and pains (especially in the back and legs). Headache is prominent, often with photophobia and retrobulbar aching. Respiratory symptoms may be mild at first, with scratchy sore throat, substernal burning, nonproductive cough and sometimes coryza. Later, lower respiratory tract illness becomes dominant; cough can be persistent, raspy and productive and may evolve in pneumonia.

While most patients recover fully often after 1 to 2 weeks, influenza and influenza-related pneumonia are important causes of morbidity or mortality in high-risk patients. In France, seasonal influenza affects 2 to 8 million people and is responsible for 10,000 to 15,000 deaths each year.

To date, treatment of influenza is symptomatic, with the application of sanitary measures to limit transmission, and may also involve specific antiviral treatment. The antivirals available in France are neuraminidase inhibitors, oseltamivir (Tamiflu®), which is active on types A and B viruses, is available in oral form. It reduces the duration of the disease and the severity of symptoms if taken early, i.e. within 48 hours of the onset of symptoms. It also reduces the risk of complications and mortality. Another neuraminidase inhibitor, zanamivir (Relenza®) can be prescribed in hospital in case of resistance to intravenous oseltamivir. Apart from sanitary measures, the annual flu vaccination remains the most effective way to protect oneself. It helps to reduce severe forms of influenza.

However, the emergence of resistance under treatment may occurred. A recurrence of symptoms after one day or less of treatment has also been observed in some serious cases (pneumonia or hospitalization) with antivirals treatment.

Therefore, there is still a need for effective and safe prophylactic and/or therapeutic treatments of viral infections, in particular acute viral infections, such as respiratory infections.

In December 2019, a new highly contagious viral pneumonia (R0 ≈ 2.2) emerged, and the epidemic was quickly qualified by the World Health Organization (WHO) as a threat to global public health. The first patients with this unexplained pneumonia appeared in Wuhan, China. A few days later, the virus was identified as a new beta coronavirus, a single-stranded RNA-positive virus called Severe Acute Respiratory Syndrome Coronavirus 2 (SARS-CoV-2). SARS-Cov-2 is the seventh coronavirus affecting the human population and the third highly pathogenic coronavirus after the coronaviruses outbreak of severe acute respiratory syndrome (SARS-CoV-2002) first identified in 2003 and Middle East respiratory syndrome (MERS-CoV-2012) first identified in 2012.

Currently, the incidence of disease associated with SARS-Cov-2, is found in more than 200 countries and territories worldwide and according to WHO data (22 April 2020), the number of confirmed cases worldwide has reached more than 2 million individuals including 162,956 deaths. Not all people exposed to SARS-CoV-2 are infected and not all infected patients develop severe respiratory disease. According to a study of more than 1000 patients in Wuhan, SARS-CoV-2 infects all age groups equally, although children and adolescents appear to be less affected and rarely develop severe forms. This protection to infection could only be relative since the number of cases of infection identified in the youngest age groups increases considerably, probably as a consequence of the increased frequency of performed screening.

COVID-19 is a respiratory illness generally first presenting with symptoms including headache, muscle pain, and/or fatigue/tiredness followed by fever and respiratory symptoms (such as a dry cough, shortness of breath, and/or chest tightness). While the symptoms remain mild in the majority of subjects, in others they may progress to pneumonia (referred herein as COVID-19 associated pneumonia or COVID-19 pneumonia) and/or to multi-organ failure. Complications of COVID-19 include acute respiratory distress syndrome (ARDS), RNAaemia, acute cardiac injury and secondary infections (Huang et al., Lancet. 2020;395(10223):497-506). It is estimated that about 20% of subjects suffering from COVID-19 require hospitalization and about 5% require admission to intensive care unit (ICU). COVID-19 causes substantial morbidity and mortality and may place unprecedented strain on many health systems.

With no vaccine, antiviral drug, or other specific treatment available, treatment of COVID-19 remains supportive. Over 175 treatments and vaccines clinical trials are currently registered and current therapeutic strategies include antiviral agents, notably remdesivir (a nucleotide analog), lopinavir/ritonavir (a antiretroviral therapy notably used for the treatment of human immunodeficiency virus 1 (HIV-1)), chloroquine or hydroxychloroquine, and 11-6 inhibitors immunomodulatory agents such as tocilizumab. However, there is currently neither any vaccine to prevent and/or treat COVID-19 or asymptomatic infection with SARS-CoV-2, nor any therapeutic agent with a proven efficacy for preventing and/or treating COVID-19, COVID-19 associated pneumonia or COVID-19 associated acute respiratory distress syndrome (ARDS).

Therefore, there is an urgent need for effective and safe prophylactic and/or therapeutic treatments for coronavirus infections, in particular coronavirus respiratory infections causing diseases such as SARS, MERS, COVID-19 and in particular COVID-19 associated pneumonia and COVID-19 associated acute respiratory distress syndrome (ARDS).

Nicotinamide mononucleotide (NMN) is a nucleotide that is already known.

The purpose of the present invention is thus to provide an alternative to current treatments by providing Nicotinamide mononucleotide and derivatives thereof for use in the treatment and/or prevention of a viral infections, in particular of respiratory infections, such as influenza virus or coronavirus.

The Applicant surprisingly found that the Nicotinamide mononucleotide derivatives according to the invention are potent agents to treat and/or prevent viral infections, and are well tolerated.

### SUMMARY

This invention thus relates to a Compound of Formula (I), 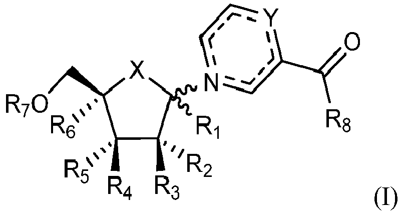 or a pharmaceutically acceptable salt or solvate thereof or prodrug thereof; wherein:
**X** is selected from O, CH₂, S, Se, CHF, CF₂ et C=CH₂;
**R₁** is selected from H, azido, cyano, C₁-C₈ alkyl, C₁-C₈ thio-alkyl, C₁-C₈ heteroalkyl and OR; wherein R is selected from H and C₁-C₈ alkyl;
**R₂**, **R₃**, **R₄** et **R₅** are independently selected from H, halogen, azido, cyano, hydroxyl, C₁-C₁₂ alkyl, C₁-C₁₂ thioalkyl, C₁-C₁₂ heteroalkyl, C₁-C₁₂ haloalkyl and OR; wherein R is selected from H, C₁-C₁₂ alkyl, C(O)(C₁-C₁₂)alkyl, C(O)NH(C₁-C₁₂)alkyl, C(O)O(C₁-C₁₂)alkyl, C(O)aryl, C(O)(C₁-C₁₂)alkyl aryl, C(O)NH(C₁-C₁₂)alkyl aryl, C(O)O(C₁-C₁₂)alkyl aryl and C(O)CHR_{AA}NH₂; wherein R_{AA} is a side chain selected from a proteinogenic amino acid;
**R₆** is selected from H, azido, cyano, C₁-C₈ alkyl, C₁-C₈ thio-alkyl, C₁-C₈ heteroalkyl and OR; wherein R is selected from H and C₁-C₈ alkyl;
**R₇** is selected from H, P(O)R₉R₁₀ et P(S)R₉R₁₀; wherein:
   R₉ and Rio are independently selected from OH, OR₁₁, NHR₁₃, NR₁₃R₁₄, C₁-C₈ alkyl, C₂-C₈ alkenyl, C₂-C₈ alkynyl, C₃-C₁₀ cycloalkyl, C₅-C₁₂ aryl, C₁-C₈ arylalkyl, C₁-C₈ alkylaryl, C₁-C₈ heteroalkyl, C₁₋C₈heterocycloalkyl, heteroaryl and NHCR_{α}R_{α'}C(O)R₁₂; wherein:
      - R₁₁ is selected from C₁-C₁₀ alkyl, C₃-C₁₀ cycloalkyl, C₅-C₁₂ aryl, C₁-C₁₀ alkylaryl, substituted C₅-C₁₂ aryl, C₁-C₁₀ heteroalkyl, C₁-C₁₀ haloalkyl, -(CH₂)ₙC(O)(C₁-C₁₅)alkyl, -(CH₂)ₙOC(O)(C₁-C₁₅)alkyl, -(CH₂)ₙOC(O)O(C₁-C₁₅)alkyl,-(CH₂)ₙSC(O)(C₁-C₁₅)alkyl, -(CH₂)ₙC(O)O(C₁-C₁₅)alkyl and -(CH₂)ₙC(O)O(C₁-C₁₅)alkyl aryl; wherein n is an integer selected from 1 to 8; and P(O)(OH)OP(O)(OH)₂;
      - R₁₂ is selected from hydrogen, C₁-C₁₀ alkyl, C₂-C₈ alkenyl, C₂-C₈ alkynyl, C₁-C₁₀ haloalkyl, C₃-C₁₀ cycloalkyl, C₃-C₁₀ cycloheteroalkyl, C₅-C₁₂ aryl, C₁-C₄ alkylaryl and C₅-C₁₂ heteroaryl; wherein said aryl or heteroaryl groups are optionally substituted by one or two groups selected from halogen, trifluoromethyl, C₁-C₆ alkyl, C₁-C₆ alkoxy and cyano;
      - R₁₃ and R₁₄ are independently selected from H, C₁-C₈ alkyl and C₁-C₈ alkyl-aryl;
      - R_{α} and R_{α'} are independently selected from an hydrogen, C₁-C₁₀ alkyl, C₂-C₁₀ alkenyl, C₂-C₁₀ alkynyl, C₃-C₁₀ cycloalkyl, C₁-C₁₀ thio-alkyl, C₁-C₁₀ hydroxylalkyl, C₁-C₁₀ alkylaryl and C₅-C₁₂ aryl, -(CH₂)₃NHC(=NH)NH₂, (1H-indol-3-yl)methyl, (1H-imidazol-4-yl)methyl and a side chain selected from a proteinogenic or non-proteinogenic amino acid; wherein said aryl groups are optionally substituted with a group selected from hydroxyl, C₁-C₁₀ alkyl, C₁-C₆ alkoxy, halogen, nitro and cyano; or
   R₉ and R₁₀ together with the phosphorus atoms to which they are attached form a 6-membered ring wherein -R₉-R₁₀- represents -CH₂-CH₂-CHR-; wherein R is selected from hydrogen, C₅-C₆ aryl and C₅-C₆ heteroaryl, wherein said aryl or heteroaryl groups are optionally substituted by one or two groups selected from halogen, trifluoromethyl, C₁-C₆ alkyl, C₁-C₆ alkoxy and cyano; or
   R9 and R10 together with the phosphorus atoms to which they are attached form a 6-membered ring wherein -R9-R10- represents -O-CH2-CH2-CHR-O-; wherein R is selected from hydrogen, C5-C6 aryl and C5-C6 heteroaryl, wherein said aryl or heteroaryl groups are optionally substituted by one or two groups selected from halogen, trifluoromethyl, C1-C6 alkyl, C1-C6 alkoxy and cyano;
**R₈** is selected from H, OR, NHR₁₃, NR₁₃R₁₄, NH-NHR₁₃, SH, CN, N₃ and halogen; wherein R₁₃ and R₁₄ are independently selected from H, C₁-C₈ alkyl and C₁-C₈ alkyl-aryl;
**Y** is selected from CH, CH₂, C(CH₃)₂ and CCH₃;
-̅-̅-̅ represents a single or double bond according to **Y**; and 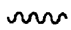 represents the alpha or beta anomer depending on the position of **R₁**,
   or a compound of formula (Ia) 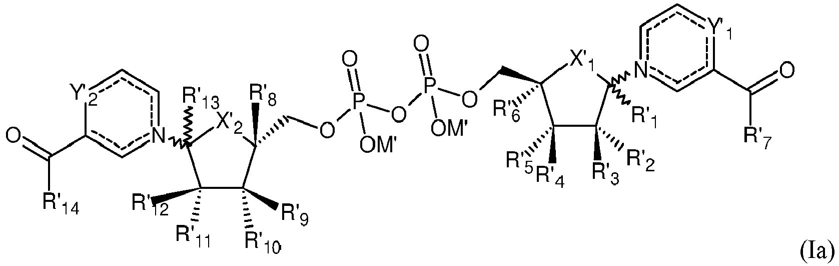 or pharmaceutically acceptable salts and/or solvates thereof or prodrugs thereof, wherein:
   - X'₁ and X'₂ are independently selected from O, CH₂, S, Se, CHF, CF₂ and C=CH₂;
   - R'₁ and R'₁₃ are independently selected from H, azido, cyano, C1-C8 alkyl, C1-C8 thio-alkyl, C1-C8 heteroalkyl and OR; wherein R is selected from H and C1-C8 alkyl;
   - R'₂, R'₃, R'₄, R'₅, R'₉, R'₁₀, R'₁₁, R'₁₂ are independently selected from H, halogen, azido, cyano, hydroxyl, C1-C12 alkyl, C1-C12 thio-alkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl or C(O)CHR_{AA}NH₂, wherein R_{AA} is a side chain selected from a proteinogenic amino acid ;
   - R'₆ and R'₈ are independently selected from H, azido, cyano, C1-C8 alkyl and OR; wherein R is selected from H and C1-C8 alkyl;
   - R'₇ and R'₁₄ are independently selected from H, OR, NHR, NRR', NH-NHR, SH, CN, N₃ and halogen; wherein R and R' are each independently selected from H, C1-C8 alkyl, C1-C8 alkyl aryl;
   - Y'₁ and Y'₂ are independently selected from CH, CH₂, C(CH₃)₂ or CCH₃;
   - M' is selected from H or a suitable counterion;
   - 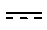 represents a single or a double bound depending on Y'₁ and Y'₂; and
   - 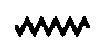 represents the alpha or beta anomer depending on the position of R'₁ and R'₁₃,
for use in the treatment and/or prevention of viral infections.

According to one embodiment, **X** represent an oxygen.

According to one embodiment, **R₁** and **R₆** each independently represents a hydrogen.

According to one embodiment, **R₂**, **R₃**, **R₄** and **R₅** each independently represents a hydrogen.

According to one embodiment, **R₂**, **R₃**, **R₄** and **R₅** each independently represents a OH.

According to one embodiment, **Y** represents a CH or a CH₂.

According to one embodiment, **R₇** represents P(O)R₉R₁₀, wherein R₉ and R₁₀ are as described in claim 1.

According to one embodiment, the compound for use according to the invention is selected from compounds **I-A** to **I-H** or pharmaceutically acceptable salts and solvates thereof or prodrugs thereof.

| **Compounds (anomeres)** | **Structure** |
|---|---|
| **I-A (beta)** | 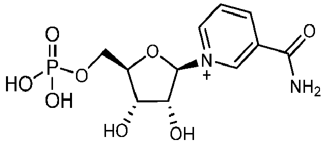 |
| **I-B (alpha)** | 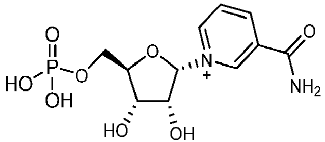 |
| **I-C (beta)** | 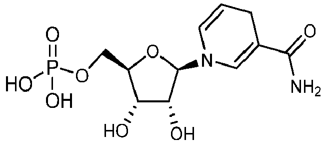 |
| **I-D (alpha)** | 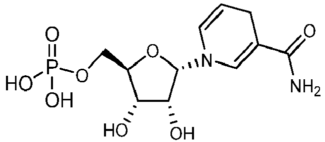 |
| **I-E (beta)** | 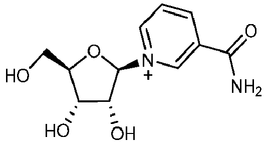 |
| **I-F (alpha)** | 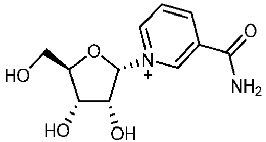 |
| **I-G (beta)** | 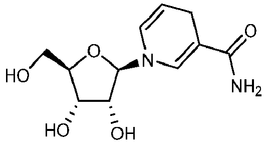 |
| **I-H (alpha)** | 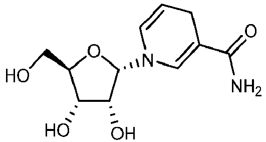 |

According to one embodiment, X'₁ and X'₂ each independently represents an oxygen.

According to one embodiment, R'₇ and R'₁₄ each independently represents a NH₂.

According to one embodiment, R'₁ and/or R'₁₃ each independently represents a hydrogen.

According to one embodiment, R'₆ and/or R'₈ each independently represents a hydrogen.

According to one embodiment, R'₂, R'₃, R'₄, R'₅, R'₉, R'₁₀, R'₁₁ and R'₁₂ each independently represents a hydrogen.

According to one embodiment, R'₂, R'₃, R'₄, R'₅, R'₉, R'₁₀, R'₁₁ and R'₁₂ each independently represents a OH.

According to one embodiment, Y'₁ and Y'₂ each independently represents a CH.

According to one embodiment, Y'₁ and Y'₂ each independently represents a CH₂.

According to one embodiment, the compound according to the invention is selected from compounds of formula Ia-A to Ia-F:

| | | |
|---|---|---|
| 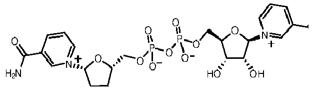 | Ia-A | Ia-D |
| | Ia-B | Ia-E |
| | Ia-C | Ia-F |

According to one embodiment, said use comprising a step of administering sequentially, simultaneously and/or separately at least another active ingredient selected from an antiviral agent, a neuraminidase inhibitor, a M2 proton channel blocker, an anti-interleukin 6, a JAK inhibitor, an interferon, a macrolide, preferably selected from the group consisting of azithromycin, clarithromycin, erythromycin, spiramycin, telithromycin, another active ingredient selected from BXT-25, chloroquine, hydroxychloroquine, brilacidin, dehydroandrographolide succinate, APN01, fingolimod, methylprednisolone, thalidomide, bevacizumab, sildenafil citrate, carrimycin, nicotine, and a mixture thereof.

According to one embodiment, the viral infection is caused by at least one virus of the genus selected from *Influenzavirus*, *Coronavirus*, *Respirovirus*, *Pneumovirus*, *Metapneumovirus, Adenovirus, Enterovirus, Rhinovirus, Hepatovirus, Erbovirus, Aphtovirus, Norovirus, Alphavirus, Rubivirus, Flavivirus, Hepacivirus, Pestivirus, Ebola-like virus, Morbillivirus, Rubulavirus, Henipavirus, Arenavirus, Orthobunyavirus, Phlebovirus, Rotavirus, Simplexvirus, Varicellovirus* or *Cytomegalovirus.*

According to one embodiment, the viral infection is a respiratory infection caused by at least one virus of the genus selected from *Influenzavirus, Coronavirus, Rhinovirus, Respirovirus, Pneumovirus* or *Metapneumovirus.*

According to one embodiment, the viral infection is a respiratory infection caused by *Influenzavirus*, preferably influenza A or influenza B.

According to one embodiment, the viral infection is a respiratory infection selected from H1N1, H3N2, H5N1, B/Yamagata/16/88-like and B/Victoria/2/87-like viruses. According to one embodiment, the coronavirus infection is selected from HCoV-229E, HCoV-NL63, HCoV-OC43, HCoV-HKU1, MERS-CoV, SARS-CoV-1 and SARS-CoV-2, preferably from MERS-CoV, SARS-CoV-1 and SARS-CoV-2.

According to one embodiment, the coronavirus infection is a SARS-CoV-2 infection causing coronavirus disease 2019 (COVID-19).

According to one embodiment, the coronavirus infection is a SARS-CoV-2 infection causing COVID-19 associated pneumonia.

According to one embodiment, the coronavirus infection is a SARS-CoV-2 infection causing COVID-19 associated acute respiratory distress syndrome (ARDS).

The invention also relates to a pharmaceutical composition for use in treatment and/or prevention of viral infections comprising at least one compound for use according to the invention and at least one pharmaceutically acceptable carrier.

According to one embodiment, the pharmaceutical composition for use comprises in addition to at least one compound for use according to the invention, at least one active ingredients, selected from an antiviral agent, a neuraminidase inhibitor, a M2 proton channel blocker, an anti-interleukin 6, a JAK inhibitor, an interferon and a mixture thereof, and/or at least another active ingredient selected from an antiviral agent, a neuraminidase inhibitor, a M2 proton channel blocker, an anti-interleukin 6, a JAK inhibitor, an interferon and a mixture thereof, and/or at least another active ingredient selected from an antiviral agent; an anti-interleukin 6 (anti-IL6) agent; a Janus-associated kinase (JAK) inhibitor; an interferon; a macrolide, preferably selected from the group consisting of azithromycin, clarithromycin, erythromycin, spiramycin, telithromycin, another active ingredient selected from BXT 25, chloroquine, hydroxychloroquine, brilacidin, dehydroandrographolide succinate, APN01, fingolimod, methylprednisolone, thalidomide, bevacizumab, sildenafil citrate, carrimycin and nicotine; and a mixture thereof..

The present invention further relates to a method for preparing compounds of formula la, comprising the following steps:
1) mono-phosphorylation of a compound of formula Xa, 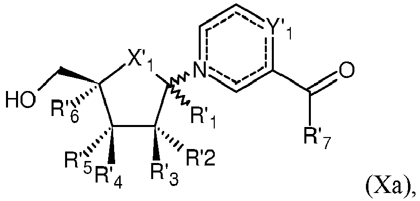 wherein:
   X'₁, R'₁, R'₂, R'₃, R'₄, R'₅, R'₆, R'₇, Y'₁, -̅-̅-̅ and 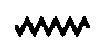 are as defined in claim 1,
   to give compound of formula XIa, 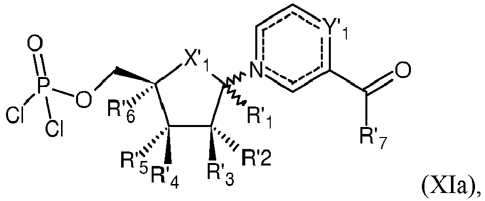 wherein:
   X'₁, R'₁, R'₂, R'₃, R'₄, R'₅, R'₆, R'₇, Y'₁, -̅-̅-̅ and 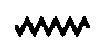 are as defined in claim 1;
2) hydrolysis of compound of formula XIa obtained in step 1), to give compound of formula XIIa 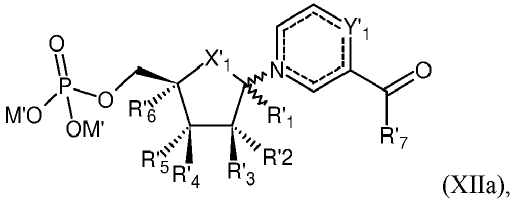 wherein: X'₁, R'₁, R'₂, R'₃, R'₄, R'₅, R'₆, R'₇, Y'₁, -̅-̅-̅ and 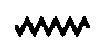 are as defined in claim 1;
3) reacting compound of formula XIIa obtained in step 2) with compound of formula XIIIa, 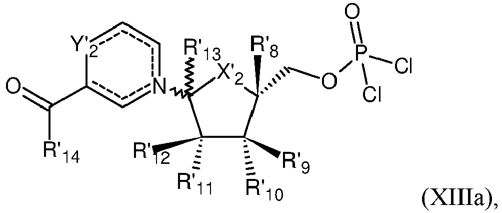 obtained as described in step 1) and wherein:
   X'₂, R'₈, R'₉, R'₁₀, R'₁₁, R'₁₂, R'₁₃, R'₁₄, Y'₂, -̅-̅-̅ and 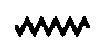 are as defined in claim 1,
   to give compound of formula Ia.

According to one embodiment, the method further comprises a step of reducing the compound of formula Ia obtained in step 3), to give the compound of formula Ia, wherein Y'₁ and Y'₂ each independently represents a CH₂.

### DEFINITIONS

The definitions and explanations below are for the terms as used throughout the entire application, including both the specification and the claims.

When describing the compounds of the invention, the terms used are to be construed in accordance with the following definitions, unless indicated otherwise.

Unless indicated otherwise, the nomenclature of substituents that are not explicitly defined herein are arrived at by naming the adjacent functionality toward the point of attachment followed by the terminal portion of the functionality. For example, the substituent "arylalkyl" refers to the group -(aryl)-(alkyl).

In the present invention, the following terms have the following meanings:
The term "**alkyl**" by itself or as part of another substituent refers to a hydrocarbyl radical of Formula CₙH₂ₙ₊₁ wherein n is a number greater than or equal to 1. Generally, alkyl groups of this invention comprise from 1 to 12 carbon atoms, preferably from 1 to 8 carbon atoms, more preferably from 1 to 6 carbon atoms, still more preferably 1 to 2 carbon atoms. Alkyl groups may be linear or branched and may be substituted as indicated herein. Suitable alkyl groups include methyl, ethyl, n-propyl, i-propyl, n- butyl, i-butyl, s-butyl and t-butyl, pentyl and its isomers (e.g. n-pentyl, iso-pentyl), and hexyl and its isomers (e.g. n-hexyl, iso-hexyl). Preferred alkyl groups include methyl, ethyl, n-propyl, i-propyl, n-butyl, i-butyl, s-butyl, t-butyl, n-pentyl, n-hexyl, n-heptyl, n-octyl, n-nonyl and n-decyl. Saturated branched alkyls include, without being limited to, isopropyl, sec-butyl, isobutyl, tert-butyl, isopentyl, 2-methylbutyl, 3-methylbutyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimtheylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, 3,3-diethylhexyl.

Suitable alkyl groups include methyl, ethyl, n-propyl, i-propyl, n-butyl, i-butyl, s-butyl and t-butyl, pentyl and its isomers (e.g. n-pentyl, iso-pentyl), hexyl and its isomers (e.g. n-hexyl, isohexyl), heptyl and its isomers (e.g. heptyl-heptyl, iso-heptyl), octyl and its isomers (e.g. n-octyl, iso-octyl), nonyl and its isomers (e.g. n-nonyl, iso-nonyl), decyl and its isomers (e.g. n-decyl, iso-decyl), undecyl and its isomers, dodecyl and its isomers. Preferred alkyl groups are methyl, ethyl, n-propyl, i-propyl, n-butyl, i-butyl, s-butyl and t-butyl. Cx-Cy-alkyl refers to alkyl groups which comprise x to y carbon atoms.

When the suffix "**ene**" ("**alkylene**") is used in conjunction with an alkyl group, this is intended to mean the alkyl group as defined herein having two single bonds as points of attachment to other groups. The term "alkylene" includes methylene, ethylene, methylmethylene, propylene, ethylethylene, and 1,2- dimethylethylene.

The term "**alkenyl**" as used herein refers to an unsaturated hydrocarbyl group, which may be linear or branched, comprising one or more carbon-carbon double bonds. Suitable alkenyl groups comprise between 2 and 12 carbon atoms, preferably between 2 and 8 carbon atoms, still more preferably between 2 and 6 carbon atoms. Examples of alkenyl groups are ethenyl, 2- propenyl, 2-butenyl, 3-butenyl, 2-pentenyl and its isomers, 2-hexenyl and its isomers, 2,4-pentadienyl and the like.

The term "**alkynyl**" as used herein refers to a class of monovalent unsaturated hydrocarbyl groups, wherein the unsaturation arises from the presence of one or more carbon-carbon triple bonds. Alkynyl groups typically, and preferably, have the same number of carbon atoms as described above in relation to alkenyl groups. Non limiting examples of alkynyl groups are ethynyl, 2- propynyl, 2-butynyl, 3-butynyl, 2-pentynyl and its isomers, 2-hexynyl and its isomers-and the like.

The term "**aryl**" as used herein refers to a polyunsaturated, aromatic hydrocarbyl group having a single ring (i.e. phenyl) or multiple aromatic rings fused together (e.g. naphtyl) or linked covalently, typically containing 5 to 12 atoms; preferably 6 to 10, wherein at least one ring is aromatic. The aromatic ring may optionally include one to two additional rings (either cycloalkyl, heterocyclyl or heteroaryl) fused thereto. Aryl is also intended to include the partially hydrogenated derivatives of the carbocyclic systems enumerated herein. Non- limiting examples of aryl comprise phenyl, biphenylyl, biphenylenyl, 5- or 6- tetralinyl, naphthalen-1- or -2-yl, 4-, 5-, 6 or 7-indenyl, 1- 2-, 3-, 4- or 5-acenaphtylenyl, 3-, 4- or 5-acenaphtenyl, 1- or 2-pentalenyl, 4- or 5-indanyl, 5-, 6-, 7- or 8-tetrahydronaphthyl, 1,2,3,4-tetrahydronaphthyl, 1,4-dihydronaphthyl, 1-, 2-, 3-, 4- or 5-pyrenyl.

The term "**cycloalkyl**" as used herein is a cyclic alkyl group, that is to say, a monovalent, saturated, or unsaturated hydrocarbyl group having 1 or 2 cyclic structures. Cycloalkyl includes monocyclic or bicyclic hydrocarbyl groups. Cycloalkyl groups may comprise 3 or more carbon atoms in the ring and generally, according to this invention comprise from 3 to 10, more preferably from 3 to 8 carbon atoms still more preferably from 3 to 6 carbon atoms. Examples of cycloalkyl groups include but are not limited to cyclopropyl, cyclobutyl, cyclopentyl, cyclohexyl, with cyclopropyl being particularly preferred.

The term "**halo**" or "**halogen**" means fluoro, chloro, bromo, or iodo. Preferred halo groups are fluoro and chloro.

The term "**haloalkyl**" alone or in combination, refers to an alkyl radical having the meaning as defined above wherein one or more hydrogens are replaced with a halogen as defined above. Non-limiting examples of such haloalkyl radicals include chloromethyl, 1-bromoethyl, fluoromethyl, difluoromethyl, trifluoro methyl, 1,1,1-trifluoroethyl and the like. Cₓ-C_{y}-haloalkyl and Cₓ-C_{y}-alkyl are alkyl groups which comprise x to y carbon atoms. Preferred haloalkyl groups are difluoromethyl and trifluoromethyl.

Where at least one carbon atom in an aryl group is replaced with a heteroatom, the resultant ring is referred to herein as a **heteroaryl ring.**

The term "**heteroalkyl**" means an alkyl group as defined above in which one or more carbon atoms are replaced by a heteroatom selected from oxygen, nitrogen and sulfur atoms. In heteroalkyl groups, the heteroatoms are linked along the alkyl chain only to carbon atoms, i.e. each heteroatom is separated from any other heteroatom by at least one carbon atom. However, the nitrogen and sulphur heteroatoms may optionally be oxidised and the nitrogen heteroatoms may optionally be quaternised. A heteroalkyl is bonded to another group or molecule only through a carbon atom, i.e. the bonding atom is not selected from the heteroatoms included in the heteroalkyl group.

The term "**heteroaryl**" as used herein by itself or as part of another group refers but is not limited to 5 to 12 carbon-atom aromatic rings or ring systems containing 1 to 2 rings which are fused together or linked covalently, typically containing 5 to 6 atoms; at least one of which is aromatic, in which one or more carbon atoms in one or more of these rings is replaced by oxygen, nitrogen and/or sulfur atoms where the nitrogen and sulfur heteroatoms may optionally be oxidized and the nitrogen heteroatoms may optionally be quaternized. Such rings may be fused to an aryl, cycloalkyl, heteroaryl or heterocyclyl ring. Non-limiting examples of such heteroaryl, include: furanyl, thiophenyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, imidazo[2, 1-b] [1,3] thiazolyl, thieno [3,2-b] furanyl, thieno [3,2-b] thiophenyl, thieno[2,3-d][1,3]thiazolyl, thieno[2,3-d]imidazolyl, tetrazolo[1,5-a]pyridinyl, indolyl, indolizinyl, isoindolyl, benzofuranyl, isobenzofuranyl, benzothiophenyl, isobenzothiophenyl, indazolyl, benzimidazolyl, 1,3-benzoxazolyl, 1,2- benzisoxazolyl, 2,1-benzisoxazolyl, 1,3-benzothiazolyl, 1,2-benzoisothiazolyl, 2,1-benzoisothiazolyl, benzotriazolyl, 1,2,3-benzoxadiazolyl, 2,1,3-benzoxadiazolyl, 1 ,2,3-benzothiadiazolyl, 2, 1 ,3-benzothiadiazolyl, thienopyridinyl, purinyl, imidazo[1,2-a]pyridinyl, 6-oxo-pyridazin-1(6H)-yl, 2- oxopyridin-1(2H)-yl, 6-oxo-pyridazin-1(6H)-yl, 2-oxopyridin-1(2H)-yl, 1,3- benzodioxolyl, quinolinyl, isoquinolinyl, cinnolinyl, quinazolinyl, quinoxalinyl.

Where at least one carbon atom in a cycloalkyl group is replaced with a heteroatom, the resultant ring is referred to herein as "**heterocycloalkyl**" or "**heterocyclyl**".

The terms "**heterocyclyl**", "**heterocycloalkyl**" or "**heterocyclo**" as used herein by itself or as part of another group refer to non-aromatic, fully saturated or partially unsaturated cyclic groups (for example, 3 to 7 member monocyclic, 7 to 11 member bicyclic, or containing a total of 3 to 10 ring atoms) which have at least one heteroatom in at least one carbon atom-containing ring. Each ring of the heterocyclic group containing a heteroatom may have 1, 2, 3 or 4 heteroatoms selected from nitrogen, oxygen and/or sulfur atoms, where the nitrogen and sulfur heteroatoms may optionally be oxidized and the nitrogen heteroatoms may optionally be quaternized. Any of the carbon atoms of the heterocyclic group may be substituted by oxo (for example piperidone, pyrrolidinone). The heterocyclic group may be attached at any heteroatom or carbon atom of the ring or ring system, where valence allows. The rings of multi- ring heterocycles may be fused, bridged and/or joined through one or more spiro atoms. Non limiting exemplary heterocyclic groups include oxetanyl, piperidinyl, azetidinyl, 2-imidazolinyl, pyrazolidinyl imidazolidinyl, isoxazolinyl, oxazolidinyl, isoxazolidinyl, thiazolidinyl, isothiazolidinyl, piperidinyl, 3H- indolyl, indolinyl, isoindolinyl, 2-oxopiperazinyl, piperazinyl, homopiperazinyl, 2-pyrazolinyl, 3-pyrazolinyl, tetrahydro-2H-pyranyl, 2H-pyranyl, 4H-pyranyl, 3,4-dihydro-2H-pyranyl, 3-dioxolanyl, 1,4-dioxanyl, 2,5-dioximidazolidinyl, 2-oxopiperidinyl, 2-oxopyrrolodinyl, indolinyl, tetrahydropyranyl, tetrahydrofuranyl, tetrahydroquinolinyl, tetrahydroisoquinolin-1-yl, tetrahydroisoquinolin-2-yl, tetrahydroisoquinolin-3-yl, tetrahydroisoquinolin-4-yl, thiomorpholin-4-yl, thiomorpholin-4-ylsulf oxide, thiomorpholin-4-ylsulfone, 1,3- dioxolanyl, 1,4-oxathianyl, 1H-pyrrolizinyl, tetrahydro-1,1-dioxothiophenyl, N- formylpiperazinyl, and morpholin-4-yl.

The term "**non-proteinogenic amino acid**" as used herein refers to an amino acid not naturally encoded or found in the genetic code of living organism. Non limiting examples of Non-proteinogenic amino acid are ornithine, citrulline, argininosuccinate, homoserine, homocysteine, cysteine-sulfinic acid, 2-aminomuconic acid, δ-aminolevulinic acid, β-alanine, cystathionine, γ-aminobutyrate, DOPA, 5-hydroxytryptophan, D-serine, ibotenic acid, α-aminobutyrate, 2-aminoisobutyrate, D-leucine, D-valine, D-alanine or D-glutamate .

The term "**proteinogenic amino acid**" as used herein refers to an amino acid that is incorporated into proteins during translation of messenger RNA by ribosomes in living organisms, i.e. Alanine (ALA), Arginine (ARG), Asparagine (ASN), Aspartate (ASP), Cysteine (CYS), Glutamate (glutamic acid) (GLU), Glutamine (GLN), Glycine (GLY), Histidine (HIS), Isoleucine (ILE), Leucine (LEU), Lysine (LYS), Methionine (MET), Phenylalanine (PHE), Proline (PRO), Pyrrolysine (PYL), Selenocysteine (SEL), Serine (SER), Threonine (THR), Tryptophan (TRP), Tyrosine (TYR) or Valine (VAL).

The term "**prodrug**" as used herein means the pharmacologically acceptable derivatives of compounds of formula (I) such as esters whose in vivo biotransformation product is the active drug. Prodrugs are characterized by increased bio-availability and are readily metabolized into the active compounds in vivo. Suitable prodrugs for the purpose of the invention include carboxylic esters, in particular alkyl esters, aryl esters, acyloxyalkyl esters, and dioxolene carboxylic esters; ascorbic acid esters.

The term "**substituent**" or "**substituted**" means that a hydrogen radical on a compound or group is replaced by any desired group which is substantially stable under the reaction conditions in an unprotected form or when protected by a protecting group. Examples of preferred substituents include, without being limited to, halogen (chloro, iodo, bromo, or fluoro); alkyl; alkenyl; alkynyl, as described above; hydroxy; alkoxy; nitro; thiol; thioether; imine; cyano; amido; phosphonato; phosphine; carboxyl; thiocarbonyl; sulfonyl; sulfonamide; ketone; aldehyde; ester; oxygen (-O); haloalkyl (*e.g*., trifluoromethyl); cycloalkyl, which may be monocyclic or fused or non-fused polycyclic (*e.g*., cyclopropyl, cyclobutyl, cyclopentyl, or cyclohexyl), or a heterocycloalkyl, which may be monocyclic or fused or non-fused polycyclic (*e.g*., pyrrolidinyl, piperidinyl, piperazinyl, morpholinyl, or thiazinyl), monocyclic or fused or non-fused polycyclic aryl or heteroaryl (*e.g*., phenyl, naphthyl, pyrrolyl, indolyl, furanyl, thiophenyl, imidazolyl, oxazolyl, isoxazolyl, thiazolyl, triazolyl, tetrazolyl, pyrazolyl, pyridyl, quinolinyl, isoquinolinyl, acridinyl, pyrazinyl, pyridazinyl, pyrimidinyl, benzimidazolyl, benzothiophenyl, or benzofuranyl); amino (primary, secondary, or tertiary); CO₂CH₃; CONH₂; OCH₂CONH₂; NH₂; SO₂NH₂; OCHF₂; CF₃; OCF₃; and such moieties may also be optionally substituted by a fused-ring structure or bridge, for example -OCH₂O-. These substituents may optionally be further substituted with a substituent selected from such groups. In certain embodiments, the term "substituent" or the adjective "substituted" refers to a substituent selected from the group consisting of an alkyl, an alkenyl, an alkynyl, an cycloalkyl, an cycloalkenyl, a heterocycloalkyl, an aryl, a heteroaryl, an arylalkyl, a heteroarylalkyl, a haloalkyl, -C(O)NR₁₁R₁₂, -NR₁₃C(O)R₁₄, a halo, -OR₁₃, cyano, nitro, a haloalkoxy, -C(O)R₁₃, -NR₁₁R₁₂, -SR₁₃, -C(O)OR₁₃, -OC(O)R₁₃, -NR₁₃C(O)NR₁₁R₁₂, -OC(O)NR₁₁R₁₂, -NR₁₃C(O)OR₁₄, -S(O)rR₁₃, -NR₁₃S(O)rR₁₄, -OS(O)rR₁₄, S(O)rNR₁₁R₁₂, -O, -S, and -N-R₁₃, wherein r is 1 or 2; R₁₁ and R₁₂, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocycloalkyl, an optionally substituted aryl, an optionally substituted heteroaryl, an optionally substituted arylalkyl, or an optionally substituted heteroarylalkyl; or R₁₁ and R₁₂ taken together with the nitrogen to which they are attached is optionally substituted heterocycloalkyl or optionally substituted heteroaryl; and R₁₃ and R₁₄ for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocycloalkyl, an optionally substituted aryl, an optionally substituted heteroaryl, an optionally substituted arylalkyl, or an optionally substituted heteroarylalkyl. In certain embodiments, the term "substituent" or the adjective "substituted" refers to a solubilizing group.

The term "**active ingredient**" refers to a molecule or a substance whose administration to a subject slows down or stops the progression, aggravation, or deterioration of one or more symptoms of a disease, or condition; alleviates the symptoms of a disease or condition; cures a disease or condition. According to one embodiment, the therapeutic ingredient is a small molecule, either natural or synthetic. According to another the therapeutic ingredient is a biological molecule such as for example an oligonucleotide, a siRNA, a miRNA, a DNA fragment, an aptamer, an antibody and the like.

By "**pharmaceutically acceptable**" is meant that the ingredients of a pharmaceutical composition are compatible with each other and not deleterious to the patient thereof.

The term "**pharmaceutically acceptable excipient**" or "**pharmaceutical vehicle**" refers to an inert medium or carrier used as a solvent or diluent in which the pharmaceutically active agent is formulated and/or administered, and which does not produce an adverse, allergic or other reaction when administered to an animal, preferably a human being. This includes all solvents, dispersion media, coatings, antibacterial and antifungal agents, isotonic agents, absorption retardants and other similar ingredients. For human administration, preparations must meet standards of sterility, general safety and purity as required by regulatory agencies such as the FDA or EMA. For the purposes of the invention, "**pharmaceutically acceptable excipient**" includes all pharmaceutically acceptable excipients as well as all pharmaceutically acceptable carriers, diluents, and/or adjuvants.

The term "**pharmaceutically acceptable salts**" include the acid addition and base salts thereof. Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

Suitable base salts are formed from bases which form non-toxic salts. Examples include the aluminium, arginine, benzathine, calcium, choline, diethylamine, 2-(diethylamino)ethanol, diolamine, ethanolamine, glycine, 4-(2-hydroxyethyl)-morpholine, lysine, magnesium, meglumine, morpholine, olamine, potassium, sodium, tromethamine and zinc salts.

Hemisalts of acids and bases may also be formed, for example, hemisulphate and hemicalcium salts.

Pharmaceutically acceptable salts of compounds of Formula (I) may be prepared by one or more of these methods:
(i) by reacting the compound of Formula (I) with the desired acid;
(ii) by reacting the compound of Formula (I) with the desired base;
(iii) by removing an acid- or base-labile protecting group from a suitable precursor of the compound of Formula (I) or by ring-opening a suitable cyclic precursor, *e.g*., a lactone or lactam, using the desired acid; and/or
(iv) by converting one salt of the compound of Formula (I) to another by reaction with an appropriate acid or by means of a suitable ion exchange column.

All these reactions are typically carried out in solution. The salt may precipitate from solution and be collected by filtration or may be recovered by evaporation of the solvent. The degree of ionization in the salt may vary from completely ionized to almost non-ionized.

Although generally, with respect to the salts of the compounds of the invention, pharmaceutically acceptable salts are preferred, it should be noted that the invention in its broadest sense also included non-pharmaceutically acceptable salts, which may for example be used in the isolation and/or purification of the compounds of the invention. For example, salts formed with optically active acids or bases may be used to form diastereoisomeric salts that can facilitate the separation of optically active isomers of the compounds of Formula I above.

The term "**solvate**" is used herein to describe a molecular complex comprising a compound of the invention and contains stoichiometric or sub-stoichiometric amounts of one or more pharmaceutically acceptable solvent molecule, such as ethanol. The term '**hydrate**' refers to when said solvent is water.

The term "**administration**", or a variant thereof (*e.g*., "**administering**"), means providing the active agent or active ingredient, alone or as part of a pharmaceutically acceptable composition, to the patient in whom/which the condition, symptom, or disease is to be treated or prevented.

The term "**human**" refers to a subject of both genders and at any stage of development (*i.e*., neonate, infant, juvenile, adolescent, adult).

The term "**patient**" refers to a warm-blooded animal, more preferably a human, who/which is awaiting the receipt of, or is receiving medical care or is/will be the object of a medical procedure.

The terms "**treat**", "**treating**" and "**treatment**", as used herein, are meant to include alleviating, attenuating or abrogating a condition or disease and/or its attendant symptoms.

The terms "**prevent**", "**preventing**" and "**prevention**", as used herein, refer to a method of delaying or precluding the onset of a condition or disease and/or its attendant symptoms, barring a patient from acquiring a condition or disease, or reducing a patient's risk of acquiring a condition or disease.

The term "**therapeutically effective amount**" (or more simply an "**effective amount**") as used herein means the amount of active agent or active ingredient that is sufficient to achieve the desired therapeutic or prophylactic effect in the patient to which/whom it is administered.

The bonds of an asymmetric carbon can be represented here using a solid triangle ( 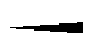 ), a dashed triangle ( 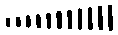 ) or a zigzag line ( 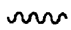 ).

### DETAILED DESCRIPTION

### Compound for use in the treatment and /or prevention of viral infections

This invention relates to a compound of Formula (I) 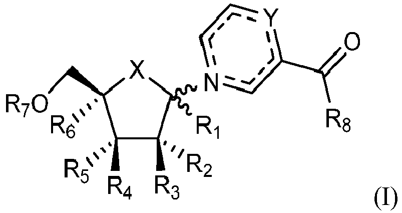 or a pharmaceutically acceptable salt or solvate thereof or prodrug thereof;
wherein:
**X** is selected from O, CH₂, S, Se, CHF, CF₂ et C=CH₂;
**R₁** is selected from H, azido, cyano, C₁-C₈ alkyl, C₁-C₈thio-alkyl, C₁-C₈ heteroalkyl and OR; wherein R is selected from H and C₁-C₈ alkyl;
**R₂**, **R₃**, **R₄** et **R₅** are independently selected from H, halogen, azido, cyano, hydroxyl, C₁-C₁₂ alkyl, C₁-C₁₂ thioalkyl, C₁-C₁₂ heteroalkyl, C₁-C₁₂ haloalkyl and OR; wherein R is selected from H, C₁-C₁₂ alkyl, C(O)(C₁-C₁₂)alkyl, C(O)NH(C₁-C₁₂)alkyl, C(O)O(C₁-C₁₂)alkyl, C(O)aryl, C(O)(C₁-C₁₂)alkyl aryl, C(O)NH(C₁-C₁₂)alkyl aryl, C(O)O(C₁-C₁₂)alkyl aryl and C(O)CHR_{AA}NH₂; wherein R_{AA} is a side chain selected from a proteinogenic amino acid;
**R₆** is selected from H, azido, cyano, C₁-C₈ alkyl, C₁-C₈thio-alkyl, C₁-C₈ heteroalkyl and OR; wherein R is selected from H and C₁-C₈ alkyl;
**R₇** is selected from H, P(O)R₉R₁₀ et P(S)R₉R₁₀; wherein:
   R₉ and R₁₀ are independently selected from OH, OR₁₁, NHR₁₃, NR₁₃R₁₄, C₁-C₈ alkyl, C₂-C₈ alkenyl, C₂-C₈ alkynyl, C₃-C₁₀ cycloalkyl, C₅-C₁₂ aryl, C₁-C₈ arylalkyl, C₁-C₈ alkylaryl, C₁-C₈ heteroalkyl, C₁-C₈ heterocycloalkyl, heteroaryl and NHCR_{α}R_{α'}C(O)R₁₂; wherein:
   - R₁₁ is selected from C₁-C₁₀ alkyl, C₃-C₁₀ cycloalkyl, C₅-C₁₂ aryl, C₁-C₁₀ alkylaryl, substituted C₅-C₁₂ aryl, C₁-C₁₀ heteroalkyl, C₁-C₁₀ haloalkyl,-(CH₂)ₙC(O)(C₁-C₁₅)alkyl, -(CH₂)ₙOC(O)(C₁-C₁₅)alkyl, -(CH₂)ₙOC(O)O(C₁-C₁₅)alkyl, -(CH₂)ₙSC(O)(C₁-C₁₅)alkyl, -(CH₂)ₙC(O)O(C₁-C₁₅)alkyl and-(CH₂)ₙC(O)O(C₁-C₁₅)alkyl aryl; wherein n is an integer selected from 1 to 8; and P(O)(OH)OP(O)(OH)₂;
   - R₁₂ is selected from hydrogen, C₁-C₁₀ alkyl, C₂-C₈ alkenyl, C₂-C₈ alkynyl, C₁-C₁₀ haloalkyl, C₃-C₁₀ cycloalkyl, C₃-C₁₀ cycloheteroalkyl, C₅-C₁₂ aryl, C₁-C₄ alkylaryl and C₅-C₁₂ heteroaryl, wherein said aryl or heteroaryl groups are optionally substituted by one or two groups selected from halogen, trifluoromethyl, C₁-C₆ alkyl, C₁-C₆ alkoxy and cyano;
   - R₁₃ and R₁₄ are independently selected from H, C₁-C₈ alkyl and C₁-C₈ alkyl-aryl;
   - R_{α} and R_{α'} are independently selected from an hydrogen, C₁-C₁₀ alkyl, C₂-C₁₀ alkenyl, C₂-C₁₀ alkynyl, C₃-C₁₀ cycloalkyl, C₁-C₁₀ thio-alkyl, C₁-C₁₀ hydroxylalkyl, C₁-C₁₀ alkylaryl and C₅-C₁₂ aryl, -(CH₂)₃NHC(=NH)NH₂, (1H-indol-3-yl)methyl, (1H-imidazol-4-yl)methyl and a side chain selected from a proteinogenic or non-proteinogenic amino acid; wherein said aryl groups are optionally substituted with a group selected from hydroxyl, C₁-C₁₀ alkyl, C₁-C₆ alkoxy, halogen, nitro and cyano; or
   R₉ and R₁₀ together with the phosphorus atoms to which they are attached form a 6-membered ring wherein -R₉-R₁₀- represents -CH₂-CH₂-CHR-; wherein R is selected from hydrogen, C₅-C₆ aryl and C₅-C₆ heteroaryl; wherein said aryl or heteroaryl groups are optionally substituted by one or two groups selected from halogen, trifluoromethyl, C₁-C₆ alkyl, C₁-C₆ alkoxy and cyano; or
   R9 and R10 together with the phosphorus atoms to which they are attached form a 6-membered ring wherein -R9-R10- represents -O-CH2-CH2-CHR-O-; wherein R is selected from hydrogen, C5-C6 aryl and C5-C6 heteroaryl, wherein said aryl or heteroaryl groups are optionally substituted by one or two groups selected from halogen, trifluoromethyl, C1-C6 alkyl, C1-C6 alkoxy and cyano;
**R₈** is selected from H, OR, NHR₁₅, NR₁₅R₁₆, NH-NHR₁₃, SH, CN, N₃ and halogen; wherein R₁₅ and R₁₆ are independently selected from H, C₁-C₈ alkyl and C₁-C₈ alkyl-aryl;
**Y** is selected from CH, CH₂, C(CH₃)₂ and CCH₃;
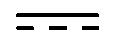 represents a single or double bond according to **Y**; and 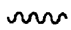
represents the alpha or beta anomer depending on the position of **R₁**,
or a compound of formula (Ia) 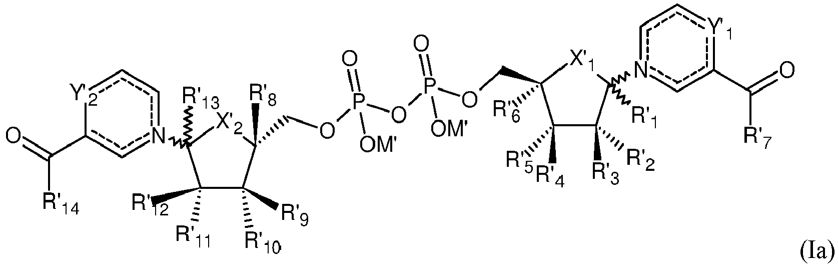 or pharmaceutically acceptable salts and/or solvates thereof or prodrugs thereof,
wherein:
- X'₁ and X'₂ are independently selected from O, CH₂, S, Se, CHF, CF₂ and C=CH₂;
- R'₁ and R'₁₃ are independently selected from H, azido, cyano, C1-C8 alkyl, C1-C8 thio-alkyl, C1-C8 heteroalkyl and OR; wherein R is selected from H and C1-C8 alkyl;
- R'₂, R'₃, R'₄, R'₅, R'₉, R'₁₀, R'₁₁, R'₁₂ are independently selected from H, halogen, azido, cyano, hydroxyl, C1-C12 alkyl, C1-C12 thio-alkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl or C(O)CHR_{AA}NH₂, wherein R_{AA} is a side chain selected from a proteinogenic amino acid ;
- R'₆ and R'₈ are independently selected from H, azido, cyano, C1-C8 alkyl and OR; wherein R is selected from H and C1-C8 alkyl;
- R'₇ and R'₁₄ are independently selected from H, OR, NHR, NRR', NH-NHR, SH, CN, N₃ and halogen; wherein R and R' are each independently selected from H, C1-C8 alkyl, C1-C8 alkyl aryl;
- Y'₁ and Y'₂ are independently selected from CH, CH₂, C(CH₃)₂ or CCH₃;
- M' is selected from H or a suitable counterion;
- 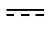 represents a single or a double bound depending on Y'₁ and Y'₂; and
- 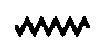 represents the alpha or beta anomer depending on the position of R'₁ and R'₁₃,
for use in the treatment and/or prevention of viral infections.

According to one embodiment, **X** is selected from O, CH₂ and S.

According to one embodiment, **R₁** is selected from hydrogen or OH. In one embodiment, **R₁** is hydrogen. In one embodiment, **R₁** is OH.

According to one embodiment, **R₂**, **R₃**, **R₄** and **R₅** are independently selected from hydrogen, halogen, hydroxyl, C₁-C₁₂ alkyl and OR; wherein R is as described herein above. In a preferred embodiment, **R₂**, **R₃**, **R₄** and **R₅** are independently selected from hydrogen, hydroxyl and OR; wherein R is as described herein above. In a more preferred embodiment **R₂**, **R₃**, **R₄** and **R₅** are independently selected from hydrogen or OH.

According to one embodiment, **R₂** and **R₃** are identical. In one embodiment, **R₂** and **R₃** are identical and represent OH. In one embodiment, **R₂** and **R₃** are identical and represent hydrogen.

According to one embodiment, **R₂** and **R₃** are different. In a preferred embodiment, **R₂** is hydrogen and **R₃** is OH. In a more preferred embodiment, **R₂** is OH and **R₃** is hydrogen.

According to one embodiment, **R₄** and **R₅** are identical. In one embodiment, **R₄** and **R₅** are identical and represent OH. In one embodiment, **R₄** and **R₅** are identical and represent hydrogen.

According to one embodiment, **R₂** and **R₃** are different. In a preferred embodiment, **R₄** is OH and **R₅** is hydrogen. In a more preferred embodiment, **R₄** is hydrogen and **R₅** is OH.

According to one embodiment, **R₃** and **R₄** are different. In one embodiment, **R₃** is OH and **R₄** is hydrogen. In one embodiment, **R₃** is hydrogen and **R₄** is OH.

According to one embodiment, **R₃** and **R₄** are identical. In a preferred embodiment, **R₃** and **R₄** are identical and represent OH. In a more preferred embodiment, **R₃** and **R₄** are identical and represent hydrogen.

According to one embodiment, **R₂** and **R₅** are different. In one embodiment, **R₂** is hydrogen and **R₅** is OH. In one embodiment, **R₂** is OH and **R₅** is hydrogen.

According to one embodiment, **R₂** and **R₅** are identical. In a preferred embodiment, **R₂** and **R₅** are identical and represent hydrogen. In a more preferred embodiment, **R₂** and **R₅** are identical and represent OH.

According to one embodiment, **R₆** is selected from hydrogen or OH. In one embodiment, **R₆** is OH. In a preferred embodiment, **R₆** is hydrogen.

According to one embodiment, **R₇** is selected from P(O)R₉R₁₀ or P(S)R₉R₁₀; wherein R₉ and R₁₀ are as described herein above. In a preferred embodiment, **R₇** is P(O)R₉R₁₀; wherein R₉ and R₁₀ are as described herein above. In a preferred embodiment, **R₇** is P(O)(OH)₂.

According to one embodiment, **R₈** is selected from H, OR, NHR₁₃ or NR₁₃R_{14;} wherein R₁₃ and R₁₄ are as described herein above. In a preferred embodiment, **R₈** is NHR₁₃; wherein R₁₃ and R₁₄ are as described herein above.

According to one embodiment, **Y** is a CH or CH₂. In one embodiment, **Y** is a CH. In one embodiment, **Y** is a CH₂.

According to one preferred embodiment, compounds of formula (I) are those wherein **X** is an oxygen.

According to a preferred embodiment, the invention relates to compounds of general Formula (II): 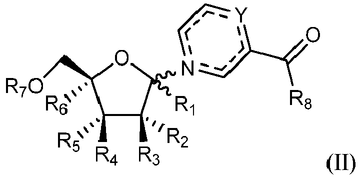 or a pharmaceutically acceptable salt or solvate thereof or prodrug thereof; wherein **R₁**, **R₂**, **R₃**, **R₄**, **R₅**, **R₆**, **R₇**, **R₈**, Y, 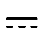 and 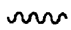 are as described herein above for compounds of formula (I).

According to one embodiment, preferred compounds of formula (I) are those wherein **R₁** is hydrogen.

According to a preferred embodiment, the invention relates to compounds of general Formula (III): 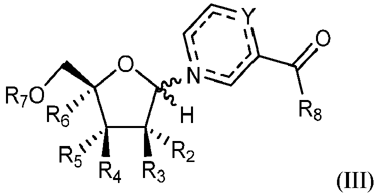 or a pharmaceutically acceptable salt or solvate thereof or prodrug thereof; wherein **R₂**, **R₃**, **R₄**, **R₅**, **R₆**, **R₇**, **R₈**, **Y**, 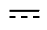 and 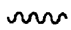 are as described herein above for compounds of formula (I).

According to one embodiment, preferred compounds of formula (I) are those wherein **R₂** is OH and **R₃** is hydrogen.

According to one embodiment, preferred compounds of formula (I) are those wherein **R₄** is hydrogen and **R₅** is OH.

According to one embodiment, preferred compounds of formula (I) are those wherein **R₃** and **R₄** are identical and represent hydrogen.

According to a preferred embodiment, the invention relates to compounds of general Formula (IV): 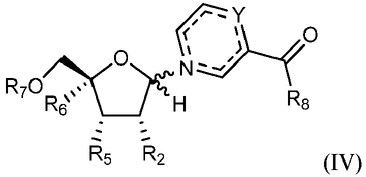 or a pharmaceutically acceptable salt or solvate thereof or prodrug thereof; wherein **R₂**, **R₅**, **R₆**, **R₇**, **R₈**, Y, 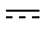 and 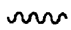 are as described herein above for compounds of formula (I).

According to one embodiment, preferred compounds of formula (I) are those wherein **R₂** and **R₅** are identical and represent OH.

According to a preferred embodiment, the invention relates to compounds of general Formula (V): 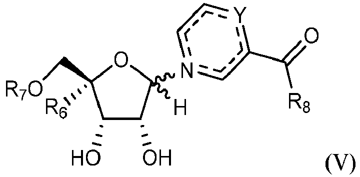 or a pharmaceutically acceptable salt or solvate thereof or prodrug thereof; wherein **R₆**, **R₇**, **R₈**, Y, 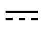 and 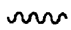 are as described herein above for compounds of formula (I).

According to one embodiment, preferred compounds of formula (I) are those wherein **R₆** is hydrogen.

According to a preferred embodiment, the invention relates to compounds of general Formula (VI): 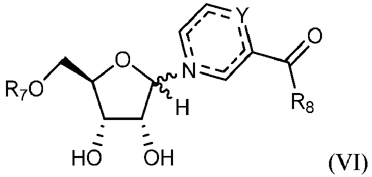 or a pharmaceutically acceptable salt or solvate thereof or prodrug thereof; wherein **R₇**, **R₈**, **Y**, 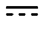 and 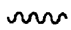 are as described herein above for compounds of formula (I).

According to one embodiment, preferred compounds of formula (I) are those wherein **R₈** is NH₂.

According to a preferred embodiment, the invention relates to compounds of general Formula (VII): 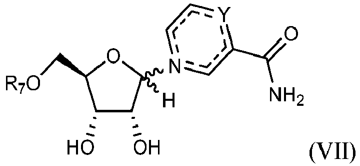 or a pharmaceutically acceptable salt or solvate thereof or prodrug thereof; wherein **R₇**, **Y**, 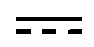 and 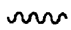 are as described herein above for compounds of formula (I).

According to one embodiment, preferred compounds of formula (I) are those wherein **Y** is CH.

According to a preferred embodiment, the invention relates to compounds of general Formula (VIII): 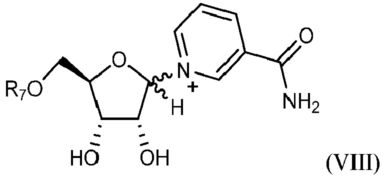 or a pharmaceutically acceptable salt or solvate thereof or prodrug thereof; wherein **R₇**, 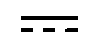 and 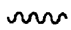 are as described herein above for compounds of formula (I).

According to one embodiment, preferred compounds of formula (I) are those wherein **Y** is CH₂.

According to a preferred embodiment, the invention relates to compounds of general Formula (IX): 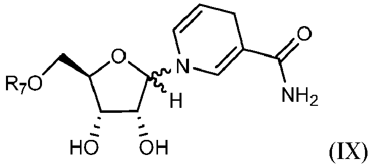 or a pharmaceutically acceptable salt or solvate thereof or prodrug thereof; wherein **R₇**, 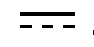 and 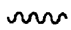 are as described herein above for compounds of formula (I).

According to one embodiment, preferred compounds of formula (I) are those wherein **R₇** is P(O)(OH)₂.

According to a preferred embodiment, the invention relates to compounds of general Formula (X): 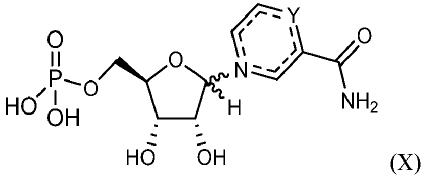 or a pharmaceutically acceptable salt or solvate thereof or prodrug thereof; wherein **Y**, 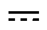 and 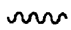 are as described herein above for compounds of formula (I).

According to one embodiment, the compound according to the invention is selected from compounds **I-A** to **I-H** from **Table 2** below or a pharmaceutically acceptable salt or solvate thereof or prodrug thereof:

**[Table 2]**

| **Compounds (anomeres)** | **Structure** |
|---|---|
| **I-A (beta)** | 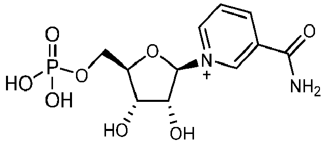 |
| **I-B (alpha)** | 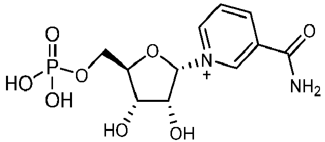 |
| **I-C (beta)** | 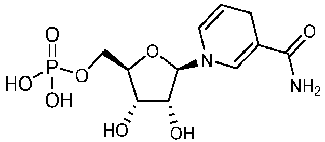 |
| **I-D (alpha)** | 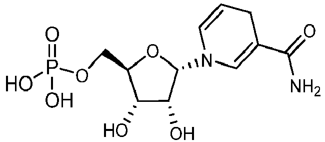 |
| **I-E (beta)** | 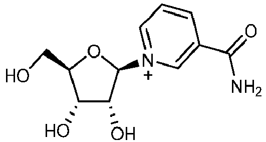 |
| **I-F (alpha)** | 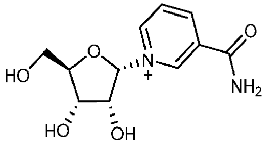 |
| **I-G (beta)** | 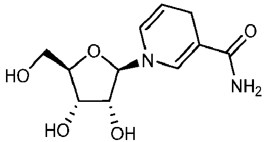 |
| **I-H (alpha)** | 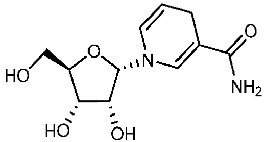 |

According to one embodiment, preferred compound of the invention are compounds **I-A to I-H** or a pharmaceutically acceptable salt or solvate thereof or prodrug thereof. According to one embodiment, more preferred compound of the invention is compounds **I-A** or a pharmaceutically acceptable salt or solvate thereof or prodrug thereof.

According to an embodiment, preferred compounds of general Formula Ia are those wherein X'₁ and X'₂ are independently selected from O, CH₂, S.

According to one embodiment, R'₇ and R'₁₄ are independently selected from H, OR, NHR and NRR' wherein R and R' are independently selected from H, C1-C8 alkyl, C1-C8 alkyl aryl. According to one embodiment, R'₇ and R'₁₄ are NHR wherein R is selected from H, C1-C8 alkyl, C1-C8 alkyl aryl.

According to one embodiment, R'₂, R'₃, R'₄, R'₅, R'₉, R'₁₀, R'₁₁, R'₁₂ are independently selected from H, halogen, hydroxyl, C1-C12 alkyl and OR. According to a preferred embodiment, R'₂, R'₃, R'₄, R'₅, R'₉, R'₁₀, R'₁₁, R'₁₂ are independently selected from H, hydroxyl and OR, wherein R is as described herein above.

According to an embodiment, preferred compounds of general Formula Ia are those wherein, R'₂, R'₃, R'₄, R'₅, R'₉, R'₁₀, R'₁₁, R'₁₂ are independently selected from H and OH.

According to one embodiment, R'₂ and R'₃ are identical. According to one embodiment, R'₂ and R'₃ are identical and represent each a OH. According to one embodiment, R'₂ and R'₃ are identical and represent each hydrogen.

According to a preferred embodiment, R'₂ and R'₃ are different. According to a preferred embodiment, R'₂ is hydrogen and R'₃ is a OH. According to a more preferred embodiment, R'₂ is a OH and R'₃ is hydrogen.

According to one embodiment, R'₄ and R'₅ are identical. According to one embodiment, R'₄ and R'₅ are identical and represent each a OH. According to one embodiment, R'₄ and R'₅ are identical and represent each hydrogen.

According to a preferred embodiment, R'₄ and R'₅ are different. According to a preferred embodiment, R'₄ is a OH and R'₅ is hydrogen. According to a more preferred embodiment, R'₄ is hydrogen and R'₅ is a OH.

According to one embodiment, R'₃ and R'₄ are identical. According to one embodiment, R'₃ and R'₄ are identical and represent each a OH. According to one embodiment, R'₃ and R'₄ are identical and represent each hydrogen.

According to a preferred embodiment, R'₃ and R'₄ are different. According to a preferred embodiment, R'₃ is a OH and R'₄ is hydrogen. According to a more preferred embodiment, R'₃ is hydrogen and R'₄ is a OH

According to one embodiment, R'₂ and R'₅ are different. According to one embodiment, R'₂ is hydrogen and R'₅ is a OH. According to one embodiment, R'₂ is a OH and R'₅ is hydrogen.

According to a preferred embodiment, R'₂ and R'₅ are identical. According to a preferred embodiment, R'₂ and R'₅ are identical and represent each hydrogen. According to a more preferred embodiment, R'₂ and R'₅ are identical and represent each a OH.

According to one embodiment, R'₉ and R'₁₀ are identical. According to one embodiment, R'₉ and R'₁₀ are identical and represent each a OH. According to one embodiment, R'₉ and R'₁₀ are identical and represent each hydrogen.

According to a preferred embodiment, R'₉ and R'₁₀ are different. According to a preferred embodiment, R'₉ is hydrogen and R'₁₀ is a OH. According to a more preferred embodiment, R'₉ is a OH and R'₁₀ is hydrogen.

According to one embodiment, R'₁₁ and R'₁₂ are identical. According to one embodiment, R'₁₁ and R'₁₂ are identical and represent each a OH. According to one embodiment, R'₁₁ and R'₁₂ are identical and represent each hydrogen.

According to a preferred embodiment, R'₁₁ and R'₁₂ are different. According to a preferred embodiment, R'₁₁ is a OH and R'₁₂ is hydrogen. According to a more preferred embodiment, R'₁₁ is hydrogen and R'₁₂ is a OH.

According to one embodiment, R'₁₀ and R'₁₁ are different. According to one embodiment, R'₁₀ is hydrogen and R'₁₁ is a OH. According to one embodiment, R'₁₀ is a OH and R'₁₁ is hydrogen.

According to a preferred embodiment, R'₁₀ and R'₁₁ are identical. According to a preferred embodiment, R'₁₀ and R'₁₁ are identical and represent each a OH. According to a more preferred embodiment, R'₁₀ and R'₁₁ are identical and represent each hydrogen.

According to one embodiment, R'₉ and R'₁₂ are different. According to one embodiment, R'₉ is hydrogen and R'₁₂ is a OH. According to one embodiment, R'₉ is a OH and R'₁₂ is hydrogen.

According to a preferred embodiment, R'₉ and R'₁₂ are identical. According to a preferred embodiment, R'₉ and R'₁₂ are identical and represent each hydrogen. According to a more preferred embodiment, R'₉ and R'₁₂ are identical and represent each a OH.

According to one embodiment, Y'₁ is CH. According to one embodiment, Y'₁ is CH₂.

According to one embodiment, Y'₂ is CH. According to one embodiment, Y'₂ is CH₂.

According to one embodiment, X'₁ and X'₂ are different and are selected from the group as described above. According to one embodiment, X'₁ and X'₂ are identical and are selected from the group as described above.

According to an embodiment, preferred compounds of general Formula Ia are those wherein X'₁ and X'₂ each independently represents an Oxygen.

According to an embodiment, preferred compounds of general Formula Ia are those wherein X'₁ and X'₂ are identical and represent each an Oxygen.

According to a preferred embodiment, among the compounds of formula la, the present invention is directed to compounds having the following formula IIa: 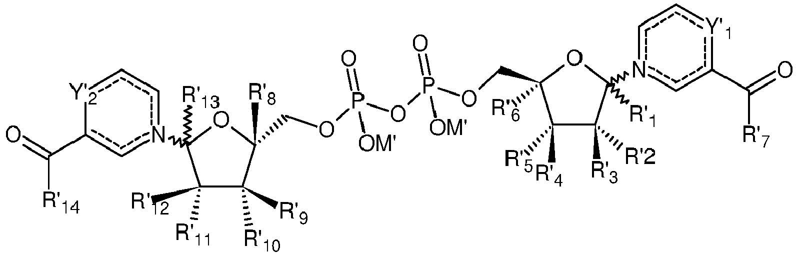 or pharmaceutically acceptable salts and/or solvates thereof or prodrugs thereof, wherein R'₁, R'₂, R'₃, R'₄, R'₅, R'₆, R'₇, R'_{9,} R'₈, R'₉, R'₁₀, R'₁₁, R'₁₂, R'₁₃, R'₁₄, Y'₁, Y'₂, M', 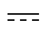 and 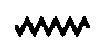 are as described above.

According to one embodiment, R'₇ and R'₁₄ are different and are selected from the group as described above. According to one embodiment, R'₇ and R'₁₄ are identical and are selected from the group as described above.

According to an embodiment, preferred compounds of general Formula Ia are those wherein R'₇ and R'₁₄ each independently represents a NH₂.

According to an embodiment, preferred compounds of general Formula Ia are those wherein R'₇ and R'₁₄ are identical and represent each a NH₂.

According to a preferred embodiment, among the compounds of formula la, the present invention is directed to compounds having the following formula IIIa: 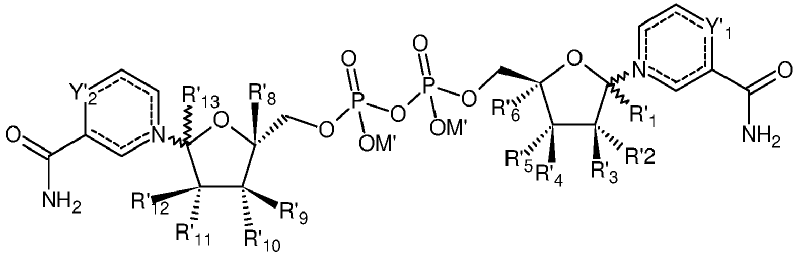 or pharmaceutically acceptable salt and/or solvates thereof or prodrugs thereof, wherein R'₁, R'₂, R'₃, R'₄, R'₅, R'₆, R'₈, R'₉, R'₁₀, R'₁₁, R'₁₂, R'₁₃, Y'₁, Y'₂, M', 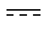 and 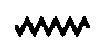 are as described above.

According to one embodiment, R'₁ and R'₁₃ are different and are selected from the group as described above. According to one embodiment, R'₁ and R'₁₃ are identical and are selected from the group as described above.

According to an embodiment, preferred compounds of general Formula Ia are those wherein R'₁ and R'₁₃ each independently represents a hydrogen.

According to an embodiment, preferred compounds of general Formula Ia are those wherein R'₁ and R'₁₃ are identical and represent each a hydrogen.

According to a preferred embodiment, among the compounds of formula la, the present invention is directed to compounds having the following formula IVa: 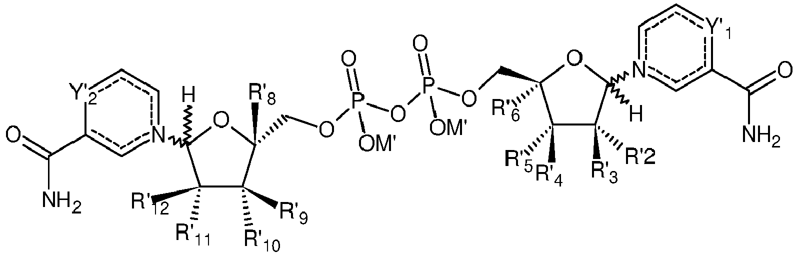 or pharmaceutically acceptable salt and/or solvates thereof or prodrugs thereof, wherein R'₂, R'₃, R'₄, R'₅, R'₆, R'₇, R'₈, R'₉, R'₁₀, R'₁₁, R'₁₂, Y'₁, Y'₂, M', 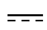 and 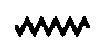 are as described above.

According to one embodiment, R'₆ and R'₈ are different and are selected from the group as described above. According to one embodiment, R'₆ and R'₈ are identical and are selected from the group as described above.

According to an embodiment, preferred compounds of general Formula Ia are those wherein R'₆ and R'₈ each independently represents a hydrogen.

According to an embodiment, preferred compounds of general Formula Ia are those wherein R'₆ and R'₈ are identical and represent each a hydrogen.

According to a preferred embodiment, among the compounds of formula la, the present invention is directed to compounds having the following formula Va: 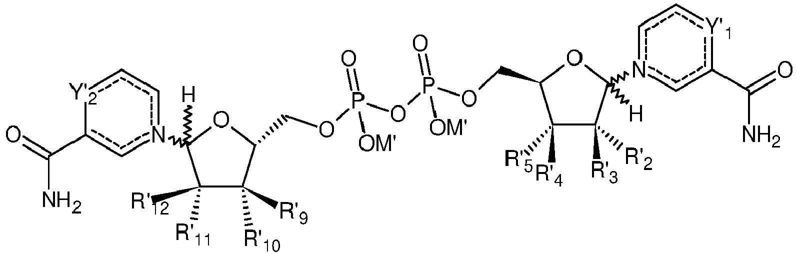 or pharmaceutically acceptable salt and/or solvates thereof or prodrugs thereof, wherein R'₂, R'₃, R'₄, R'₅, R'₇, R'₉, R'₁₀, R'₁₁, R'₁₂, Y'₁, Y'₂, M', 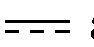 and 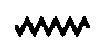 are as described above.

According to one embodiment, R'₃, R'₄, R'₁₀ and R'₁₁ are different and are selected from the group as described above. According one embodiment, R'₃, R'₄, R'₁₀ and R'₁₁ are identical and are selected from the group as described above.

According to an embodiment, preferred compounds of general Formula Ia are those wherein R'₃, R'₄, R'₁₀ and R'₁₁ each independently represents a hydrogen.

According to an embodiment, preferred compounds of general Formula Ia are those wherein R'₃, R'₄, R'₁₀, R'₁₁ are identical and represent each a H.

According to a preferred embodiment, among the compounds of formula la, the present invention is directed to compounds having the following formula VIa: 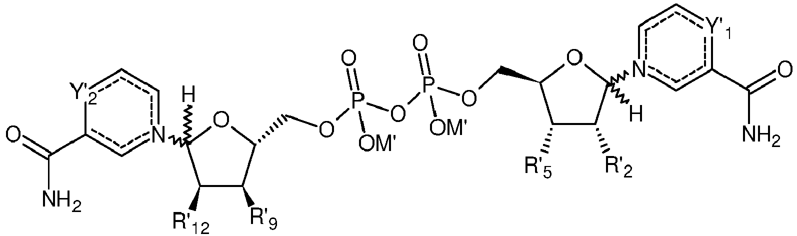 or pharmaceutically acceptable salt and/or solvates thereof or prodrugs thereof, wherein R'₂, R'₅, R'₇, R'₉, R'₁₂, Y'₁, Y'₂, M', 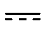 and 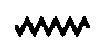 are as described above.

According to one embodiment, R'₂, R'₅, R'₉ and R'₁₂ are different and are selected from the group as described above. According one embodiment, R'₂, R'₅, R'₉ and R'₁₂ are identical and are selected from the group as described above.

According to an embodiment, preferred compounds of general Formula Ia are those wherein R'₂, R'₅, R'₉ and R'₁₂ each independently represents a OH.

According to an embodiment, preferred compounds of general Formula Ia are those wherein R'₂, R'₅, R'₉, R'₁₂ are identical and represent each a OH.

According to a preferred embodiment, among the compounds of formula la, the present invention is directed to compounds having the following formula VIIa: 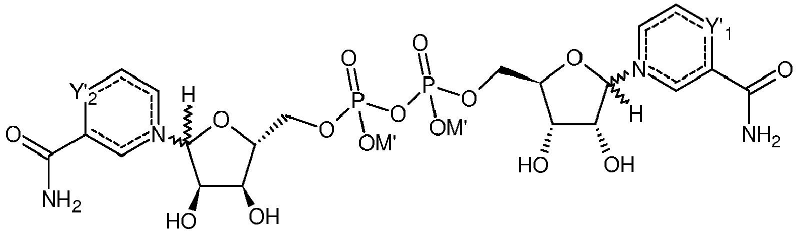 or pharmaceutically acceptable salt and/or solvates thereof or prodrugs thereof, wherein Y'₁, Y'₂, M', 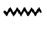 and 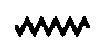 are as described above.

According to one embodiment, Y'₁ and Y'₂ are different. According to a preferred embodiment, Y'₁ and Y'₂ are identical.

According to an embodiment, preferred compounds of general Formula Ia are those wherein Y'₁ and Y'₂ each independently represents a CH.

According to an embodiment, preferred compounds of general Formula Ia are those wherein Y'₁ and Y'₂ are identical and represent each a CH.

According to a preferred embodiment, among the compounds of formula la, the present invention is directed to compounds having the following formula VIIIa: 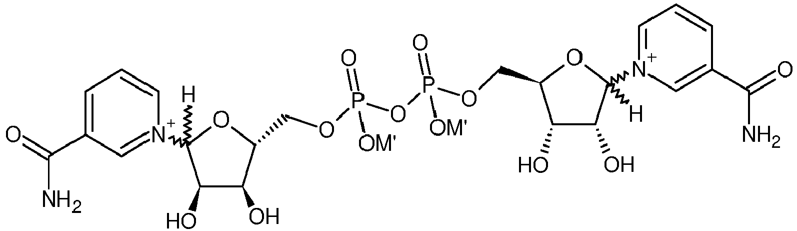 or pharmaceutically acceptable salt and/or solvates thereof or prodrugs thereof, wherein M' and 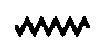 are as described above.

According to an embodiment, preferred compounds of general Formula Ia are those wherein Y'₁ and Y'₂ each independently represents a CH₂.

According to an embodiment, preferred compounds of general Formula Ia are those wherein Y'₁ and Y'₂ are identical and represent each a CH₂.

According to a preferred embodiment, among the compounds of formula Ia, the present invention is directed to compounds having the following formula IXa: 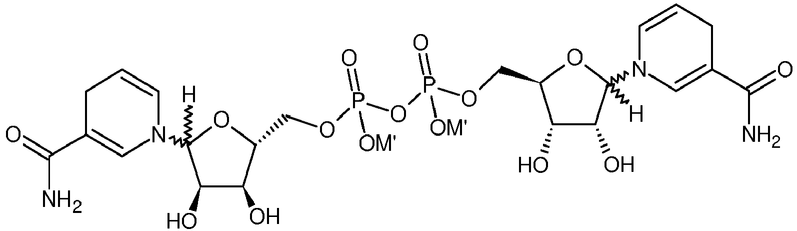 or pharmaceutically acceptable salt and/or solvates thereof or prodrugs thereof, wherein M' and 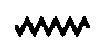 are as described above.

According to one embodiment, preferred compounds of the invention are compounds Ia-A to Ia-F, listed in table 1:

**Table 1**

| **Cpd n° (anomers)** | **Structure** |
|---|---|
| **Ia-A (beta, beta).** | 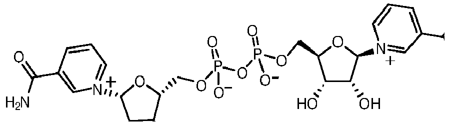 |
| **Ia-B (beta, alpha)** | |
| **Ia-C (alpha, alpha)** | |
| **Ia-D (beta, beta)** | |
| **Ia-E (beta, alpha)** | |
| **Ia-F (alpha, alpha)** | |

According to one embodiment, preferred compound of the invention is compound of formula Ia-A.

According to another embodiment, preferred compound of the invention is compound of formula Ia-D.

All references to compounds of Formula (I) or (Ia) include references to salts, solvates, multi-component complexes and liquid crystals thereof. All references to compounds of Formula (I) or (Ia) include references to polymorphs and crystal habits thereof.

All references to compounds of Formula (I) or (Ia) include references to pharmaceutically acceptable prodrugs and prodrugs thereof.

### Pharmaceutical composition

This invention also relates to a pharmaceutical composition for use in the treatment and/or prevention of viral infections, comprising the compound for the use according to the invention, and at least one pharmaceutically acceptable carrier.

According to one embodiment, the pharmaceutical composition further comprises at least another active ingredient.

In one embodiment, the other active ingredient is selected from:
an antiviral agent, a neuraminidase inhibitor such as oseltamivir, zanamivir, peramivir or laninamivir; a M2 proton channel blocker such as adamantadine or remantanide;
an anti-interleukin 6 such as tocilizumab, siltuximab, sarilumab, sirukumab, clazakizumab or olokizumab; a JAK inhibitor, such as barcitinib, fedratinib or ruxolitinib; an interferon such as interferon beta-la (IFN-β-1a), interferon beta-lb (IFN-β-1b) or peginterferon beta-la; a macrolide, preferably selected from the group consisting of azithromycin, clarithromycin, erythromycin, spiramycin, telithromycin: another active ingredient selected from BXT-25, chloroquine, hydroxychloroquine, brilacidin, dehydroandrographolide succinate, APN01, fingolimod, methylprednisolone, thalidomide, bevacizumab, sildenafil citrate, carrimycin, nicotine, and a mixture thereof.

Non-limiting examples of further antiviral agents include:
polymerase inhibitors, such as favipiravir, pimodivir, baloxavir, marboxil and sofosbuvir;
protease inhibitors, such as boceprevir, simeprevir, fosamprenavir, lopinavir, ritonavir, telaprevir, tipranavir, azatanavir, nelfinavir, indinavir and saquinavir;
integrase strand transfer inhibitors, such as raltegravir, dolutegravir and elvitegravir; NS5A inhibitors, such as daclatasvir;
nucleoside reverse transcriptase inhibitors (NRTIs), such as lamivudine, adefovir, tenofovir, entecavir and emtricitabine;
nonnucleoside reverse transcriptase inhibitors (NNRTIs), such as efavirenz, nevirapine and etravirine;
purine nucleosides, such as ribavirin, valacyclovir, acyclovir and famciclovir; and mixtures thereof.

### Process

According to another aspect, the invention relates to a method for the preparation of the compound of Formula (I) as described above.

In particular, the compounds of Formula (I) disclosed herein may be prepared as described below from substrates A-E. It shall be understood by a person skilled in the art that these schemes are in no way limiting and that variations may be made without departing from the spirit and scope of this invention.

According to one embodiment, the method involves in a first step the mono-phosphorylation of a compound of formula (A), in the presence of phosphoryl chloride and a trialkyl phosphate, to yield the phophorodichloridate of formula (B), 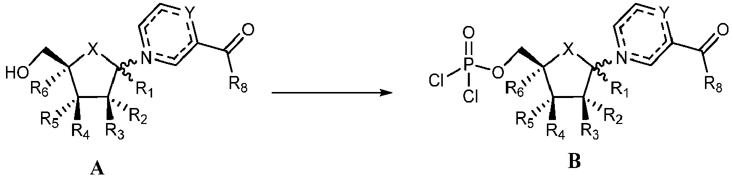 wherein X, **R₁**, **R₂**, **R₃**, **R₄**, **R₅**, **R₆**, **R₇**, **R₈**, **Y**, 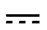 and 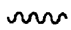 are as described herein above for compounds of formula (I).

In a second step, the phophorodichloridate of formula (B) is hydrolyzed to yield the phosphate of formula (C), 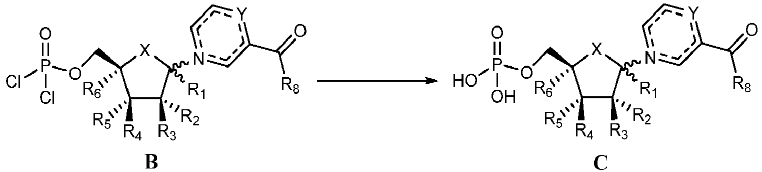 wherein X, **R₁**, **R₂**, **R₃**, **R₄**, **R₅**, **R₆**, **R₇**, **R₈**, Y, 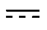 and 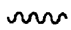 are as described herein above for compounds of formula (I).

According to one embodiment, the compound of formula (A) is synthesized using various methods known to the person skilled in the art. According to one embodiment, the compound of formula (A) is synthesized by reacting the pentose of formula (D) with a nitrogen derivative of formula (E), wherein **R, R₂**, **R₃**, **R₄**, **R₅**, **R₆**, **R₇**, **Y** are as described above for compounds of formula (I), leading to the compound of formula (A-1) which is then selectively deprotected to give the compound of formula (A), 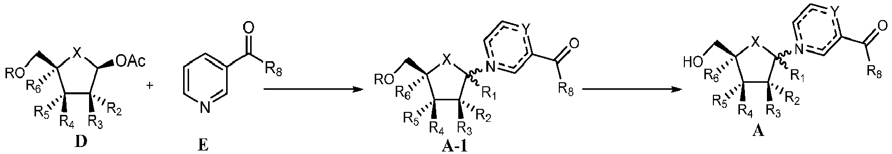 wherein X, **R₁**, **R₂**, **R₃**, **R₄**, **R₅**, **R₆**, **R₈**, **Y**, 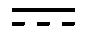 and 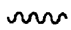 are as described herein above for compounds of formula (I).

According to one embodiment, R is an appropriate protective group known to the skilled person in the art. In one embodiment, the protecting group is selected from triarylmethyls and/or silyls. Non-limiting examples of triarylmethyl include trityl, monomethoxytrityl, 4,4'-dimethoxytrityl and 4,4',4"-trimethoxytrityl. Non-limiting examples of silyl groups include trimethylsilyl, tert-butyldimethylsilyl, triisopropylsilyl, tert-butyldiphenylsilyl, tri-iso-propylsilyloxymethyl and [2-(trimethylsilyl)ethoxy]methyl.

According to one embodiment, any hydroxyl group attached to the pentose is protected by an appropriate protective group known to the person skilled in the art.

The choice and exchange of protective groups is the responsibility of the person skilled in the art. Protective groups can also be removed by methods well known to the skilled person, for example, with an acid (e.g. mineral or organic acid), base or fluoride source. According to a preferred embodiment, the nitrogen derivative of formula (E) is coupled to the pentose of formula (D) by a reaction in the presence of a Lewis acid leading to the compound of formula (A-1). Non-limiting examples of Lewis acids include TMSOTf, BF₃.OEt₂, TiCl₄ and FeCl₃.

According to one embodiment, the method of the present invention further comprises a step of reducing the compound of formula (A) by various methods well known to the skilled person in the art, leading to the compound of formula (A') wherein is CH₂ and **R₁**, **R₂**, **R3**, **R₄**, **R₅**, **R₆**, **R₈**, **Y**, 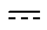 and 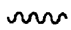 are as defined above for compounds of formula (I).

According to a specific embodiment, the present invention relates to a method for the preparation of the compounds of formula **I-A** to **I-D.**

In a first step, the nicotinamide of formula E is coupled to the ribose tetraacetate of formula **D** by a coupling reaction in the presence of a Lewis acid, resulting in the compound of formula **A-1**:

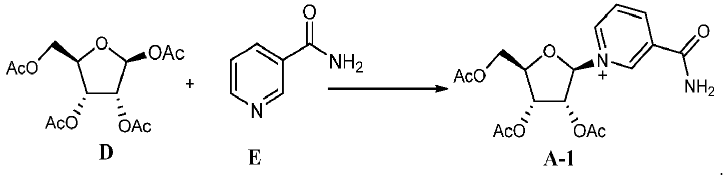

In a second step, an ammoniacal treatment of the compound of formula **A-1** is carried out, leading to the compound of formula A-2:

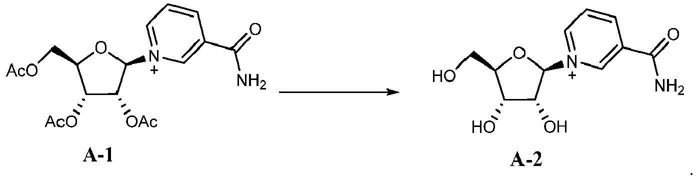

In a third step, the mono-phosphorylation of the compound of formula A-2, in the presence of phosphoryl chloride and a trialkyl phosphate, leads to the phophorodichloridate of formula A-3:

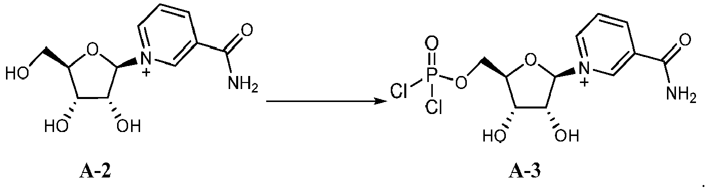

In a fourth step, the phophorodichloridate of formula A-3 is hydrolyzed to yield the compound of formula I-A:

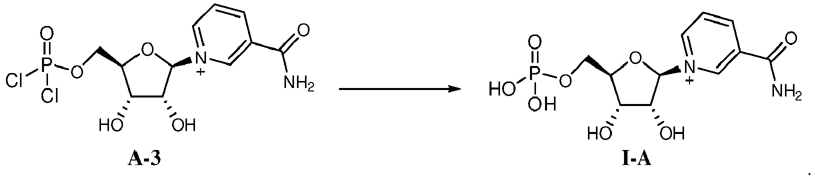

According to one embodiment, a step of reducing the compound of formula A-2 is carried out, leading to the compound of formula I-E.

The compound of formula I-E is then monophosphorylated as described in step 4 and hydrolyzed to the compound of formula I-C.

In another aspect, the invention relates to a method for preparing compounds of formula Ia as described above.

In particular, compounds of formula Ia disclosed herein can be prepared as described below from substrates Xa-XIIIa. It will be understood by one ordinary skilled in the art that these schemes are in no way limiting and that variations of detail can be made without departing from the spirit and scope of the present invention.

According to one embodiment, the invention relates to a method for preparing the compound of formula I described herein above.

The method first involves the mono-phosphorylation of a compound of formula Xa, in the presence of phosphoryl chloride in a trialkyl phosphate, to give the phophorodichloridate compound XIa, 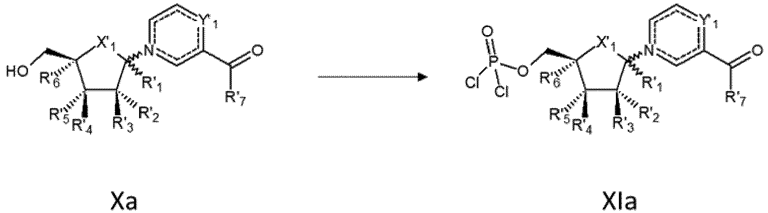 wherein X'₁, R'₁, R'₂, R'₃, R'₄, R'₅, R'₆, R'₇, Y'₁, 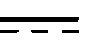 and 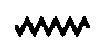 are as described herein for formula Ia.

In a second step the hydrolysis of the phophorodichloridate XIa obtained in the first step give the phosphate compound of formula XIIa, 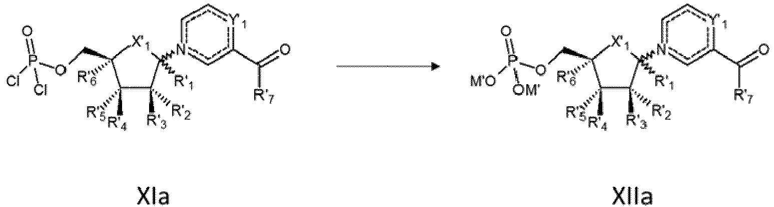 wherein X'₁, R'₁, R'₂, R'₃, R'₄, R'₅, R'₆, R'₇, Y'₁, M', 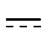 and 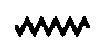 are as described herein for formula Ia.

The phosphate compound of formula XIIa obtained in the second step is then reacted, with a phophorodichloridate compound of formula XIIIa obtained as described in the first step, 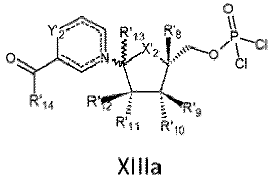 wherein X'₂, R'₈, R'₉, R'₁₀, R'₁₁, R'₁₂, R'₁₃, R'₁₄, Y'₂, 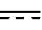 and 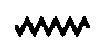 are as are as described herein for formula la, to give the compound of formula Ia as described herein.

According to one embodiment, the method of the invention further comprises a step of reducing the compound of formula la, using various methods known to those skilled in the art, to give the compound of formula I'a, wherein Y'₁ and Y'₂ are identical and represent each CH₂ and wherein X'1, X'₂, R'₁, R'₂, R'₃, R'₄, R'₅, R'₆, R'₇, R'₈, R'₉, R'₁₀, R'₁₁, R'₁₂, R'₁₃, R'₁₄, Y'₁, Y'₂, M', 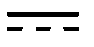 and 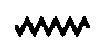 are as described herein for formula Ia. According to another embodiment, the compound of formula Xa is synthesized using various methods known to those skilled in the art. According to one embodiment, the compound of formula Xa is synthesized in two steps by first reacting the pentose of formula XIVa with the nitrogenous derivatives of formula XVa, wherein R'₁, R'₁, R'₂, R'₃, R'₄, R'₅, R'₆, R'₇, Y'₁ and R are as described herein for formula la, to give the compound of formula Xa-1, then selectively deprotected to give the compound of formula Xa. 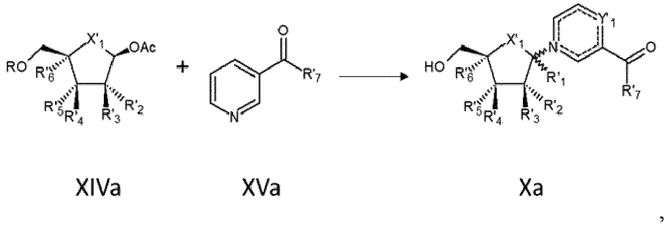 wherein X'₁, R, R'₁, R'₂, R'₃, R'₄, R'₅, R'₆, R'₇, Y'₁, 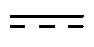 and 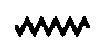 are as described herein for formula Ia.

According to one embodiment, R is an appropriate protecting group known to those skilled in the art. Examples of appropriate protecting group includes triarylmethyl and/or silyl groups. Non limiting examples of triarylmethyl includes trityl, monomethoxytrityl, 4,4'-dimethoxytrityl and 4,4',4"-trimethoxytrityl. Non limiting examples of silyl groups includes trimethylsilyl, tert-butyldimethylsilyl, triisopropylsilyl, tert-butyldiphenylsilyl, tri-iso-propylsilyloxymethyl and [2-(trimethylsilyl)ethoxy]methyl.

According to one embodiment, any hydroxy group attached to the pentose ring is protected with an appropriate protecting group known to those skilled in the art.

The selection and exchange of the protecting groups is within the skill to those skilled in the art. Any protecting groups can also be removed by methods known in the art, for example, with an acid (e.g., a mineral or an organic acid), a base or a fluoride source.

According to a preferred embodiment, the nitrogenous derivatives of formula XVa is added to the pentose XIVa via a coupling reaction in the presence of a Lewis acid to give the compound of formula Xa-1. Non limiting examples of suitable Lewis acid includes TMSOTf, BF₃.OEt₂, TiCl₄ and FeCl₃.

According to a specific embodiment, the invention relates to a method for preparing the compound of formula VIIIa, 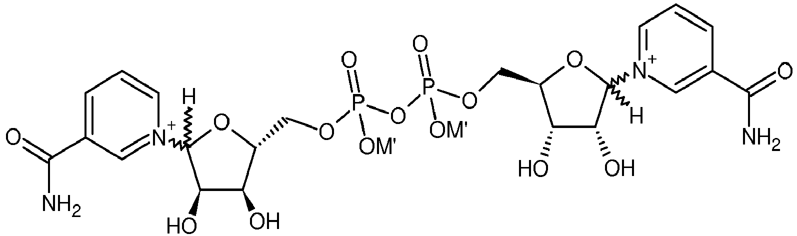 or pharmaceutically acceptable salts and/or solvates thereof or prodrugs thereof.

In a first step, the nicotinamide of formula XVa, is added to the ribose tetraacetate XIVa, via a coupling reaction in the presence of a Lewis acid, to give the compound of formula Xa-1:

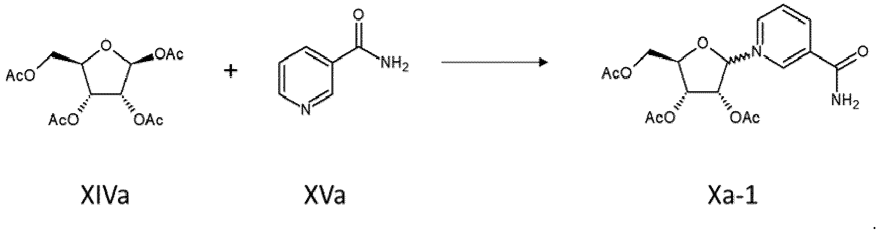

In a second step, an ammoniacal treatment of the compound of formula Xa-1 give the compound of formula Xa:

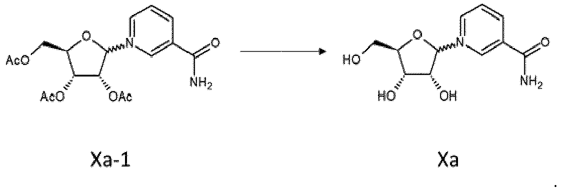

In a third step, the mono-phosphorylation of a compound of formula Xa, in the presence of phosphoryl chloride in a trialkyl phosphate, give the phophorodichloridate compound XIa:

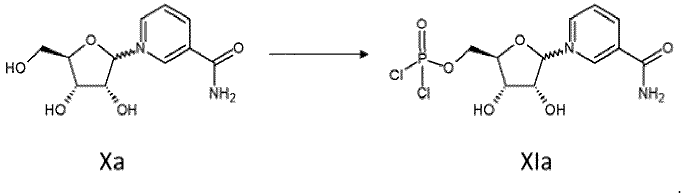

In a fourth step, the phophorodichloridate compound XIa obtained in the third step is partially hydrolyzed to give the phosphate compound of formula XIIa:

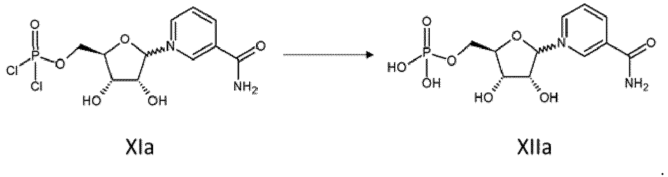

In a fifth step, the phosphate compound of formula XIIa obtained in the fourth step is then reacted, with the phophorodichloridate compound of formula XIa obtained as described in the third step, to give the compound of formula VIIIa.

According to another specific embodiment, the invention relates to a method for preparing the compound of formula IXa, 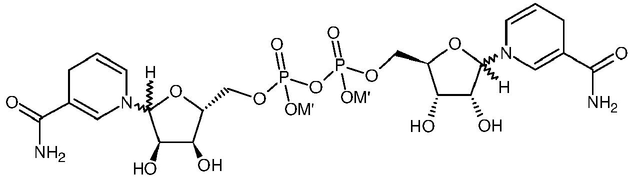 or pharmaceutically acceptable salts and/or solvates thereof or prodrugs thereof.

According to one embodiment, the compound of formula IXa is obtained from the compound of formula VIIIa, previously synthesized as described above.

In this embodiment, the compound of formula IXa is obtained by reducing the compound of formula VIIIa, using a suitable reducing agent known to those skilled in the art, to give the compound of formula IXa.

### Medical use and methods of treatment

This invention thus relates to a compound according to the invention, as described hereinabove, for use in the treatment and/or prevention of viral infections.

According to one embodiment, the compound is for use in the treatment and/or prevention of at least one viral infection.

According to one embodiment, the viral infection is caused by at least one virus selected from positive-sense ribonucleic acid (RNA) viruses, negative-sense RNA viruses, double-strand RNA viruses, single-strand deoxyribonucleic acid (DNA) viruses or double-strand DNA viruses,

According to one embodiment, the viral infection is caused by at least one virus of the genus selected from:
*Enterovirus* such as Human Enterovirus (HEV) A, HEV-B, HEV-C or HEV-D;
*Rhinovirus* such as Human rhinovirus (HRV) A or HRV-B;
*Coronavirus* such as Human coronavirus;
*Hepatovirus* such as Hepatitis virus A;
*Norovirus* such as Norwalk virus;
*Hepatite E-like virus* such as Hepatitis E virus;
*Alphavirus;*
*Rubivirus* such as rubella virus;
*Flavivirus* such as yellow fever virus, Dengue virus or West Nile virus;
*Hepacivirus* such as Hepatitis C virus;
*Pestivirus* such as bovine diarrhea virus;
*Ebola-like virus* such as Zaire Ebola virus, Cote d'Ivoire Ebola virus, Reston Ebola virus or Sudan Ebola virus;
*Respirovirus* such as Human parainfluenza virus 1 and 3;
*Morbillivirus* such as measle virus;
*Rubulavirus* such as Mumps virus or human parainfluenza virus 2, 4a and 4b;
*Henipavirus* such as Hendra virus or Nipah virus;
*Pneumovirus* such as Human respiratory syncytial virus;
*Metapneumovirus* such as Human metapneumovirus;
*Influenzavirus* such as Influenza A virus, Influenza B virus or Influenza C virus;
*Arenavirus;*
*Orthobunyavirus* such as California encephalitis virus;
*Phlebovirus* such as Rift Valley fever virus;
*Rotavirus* such as Human rotavirus A, B or C;
*Simplexvirus* such as Human herpesvirus 1 and 2;
*Varicellovirus* such as Human herpesvirus 3;
*Cytomegalovirus* such as Human herpesvirus 5;
*Lymphocryptovirus* such as Human herpesvirus 4 and 8;
*Adenovirus;*
*Papillomavirus* such as Human papillomavirus; and
*Aphtovirus* such as Foot-and-mouth disease virus.

According to one embodiment, the viral infections is a respiratory infection caused by at least one virus of the genus selected from *Influenzavirus, Rhinovirus, Coronavirus, Respirovirus, Rubulavirus, Pneumovirus, Adenovirus* or *Metapneumovirus.*

According to a preferred embodiment, the viral infections is a respiratory infection caused by *Influenzavirus*.

According to one embodiment, *Influenzavirus* is selected from influenza A, influenza B and influenza C. According to a preferred embodiment, *Influenzavirus* is selected from influenza A and influenza B.

Thus, according to a preferred embodiment, the compound is for use in the treatment and/or prevention of influenza A and/or influenza B.

According to one embodiment, influenza A is selected from H1N1, H1N2, H2N2, H3N2, H5N1, H5N2, H5N9, H7N2, H7N3, H7N7, H7N9, H9N2 and H10N7.

According to one embodiment, influenza B is selected from B/Yamagata/16/88-like and B/Victoria/2/87-like viruses.

According to a preferred embodiment, *Influenzavirus* is selected from H1N1, H3N2, H5N1, B/Yamagata/16/88-like and B/Victoria/2/87-like viruses.

According to a preferred embodiment, the compound is for use in the treatment and/or prevention of H1N1, H3N2, H5N1, B/Yamagata/16/88-like and B/Victoria/2/87-like viruses.

According to one embodiment, influenza A and/or influenza B lead to respiratory complications such as influenza A and/or influenza B associated pneumonia.

According to another embodiment, influenza A and/or influenza B lead to extra-respiratory complications such as decompensation of underlying pathologies or other extra-pulmonary complications such as Reye's syndrome associated with aspirin intake, myocarditis, pericarditis, rhabdomyolysis, Guillain Barre syndrome or encephalomyelitis.

According to one embodiment, the coronavirus infection is an alpha coronavirus infection or a beta coronavirus infection. In a preferred embodiment, the coronavirus infection is a beta coronavirus infection.

According to one embodiment, the alpha coronavirus infection is selected from human coronavirus 229E (HCoV-229E) and human coronavirus NL63 (HCoV-NL63) also sometimes known as HCoV-NH or New Haven human coronavirus.

According to one embodiment, the beta coronavirus infection is selected from human coronavirus OC43 (HCoV-OC43), human coronavirus HKU1 (HCoV-HKU1), Middle East respiratory syndrome-related coronavirus (MERS-CoV) previously known as novel coronavirus 2012 or HCoV-EMC, severe acute respiratory syndrome coronavirus (SARS-CoV) also known as SARS-CoV-1 or SARS-classic, and severe acute respiratory syndrome coronavirus (SARS-CoV-2) also known as 2019-nCoV or novel coronavirus 2019.

According to one embodiment, the coronavirus infection is selected from HCoV-229E, HCoV-NL63, HCoV-OC43, HCoV-HKU1, MERS-CoV, SARS-CoV-1 and SARS-CoV-2. In one embodiment, the coronavirus infection is selected from MERS-CoV, SARS-CoV-1 and SARS-CoV-2.

According to a preferred embodiment, the coronavirus infection is a SARS-CoV-2 infection.

According to one embodiment, the coronavirus is a MERS-CoV infection causing Middle East respiratory syndrome (MERS). According to one embodiment, the coronavirus is a SARS-CoV-1 infection causing severe acute respiratory syndrome (SARS).

According to a preferred embodiment, the coronavirus is a SARS-CoV-2 infection causing coronavirus disease 2019 (COVID-19).

Thus, according to one embodiment, the compound is for use in the treatment and/or prevention of a coronavirus infection selected from MERS-CoV, SARS-CoV-1 and SARS-CoV-2.

According to one embodiment, the compound is for use in the treatment and/or prevention of MERS, SARS and COVID-19.

According to a preferred embodiment, the compound is for use in the treatment and/or prevention of COVID-19.

According to a preferred embodiment, the compound is for use in the preexposure prophylaxis to virus, including those cited above, and preferably SARS-CoV-2.

Thus, according to one embodiment, the compound is for use in the treatment and/or prevention of respiratory or extra-respiratory complications.

According to a preferred embodiment, the compound is for use in the treatment and/or prevention of influenza A and/or influenza B associated pneumonia.

According to one embodiment, influenza A and/or influenza B associated pneumonia is a viral pneumonia causing Acute Respiratory Failure.

According to another embodiment, influenza A and/or influenza B associated pneumonia is a bacterial pneumonia due to bacterial over-infection with a bacterium of the genus selected from *Streptococcus pneumoniae, Staphylococcus aureus* and *Haemophilus influenzae.*

According to one embodiment, COVID-19 leads to respiratory complications such as COVID-19 associated pneumonia or COVID-19 associated acute respiratory distress syndrome (ARDS).

According to one embodiment, COVID-19 leads to extra-respiratory complications such as sepsis, septic shock, altered consciousness, and/or multi-organ failure.

According to one embodiment, COVID-19 associated pneumonia presents on a lung scan (such as computerized tomography (CT) scan) as hazy patches, in particular hazy patches clustering on the outer edges of the lungs. In one embodiment, COVID-19 associated pneumonia presents on a lung scan as radiological finding of ground-glass opacity abnormalities or radiological finding of a mixed pattern (combination of consolidation, ground glass opacity and reticular opacity in the presence of architectural distortion). According to one embodiment, ARDS is a form of acute lung injury (ALI) and occurs as a result of a severe pulmonary injury that causes alveolar damage heterogeneously throughout the lung.

According to one embodiment, the coronavirus infection is a SARS-CoV-2 infection causing coronavirus disease 2019 (COVID-19).

Thus, according to one embodiment, the compound is for use in the treatment and/or prevention of COVID-19 associated pneumonia or COVID-19 associated ARDS.

According to one embodiment, the coronavirus infection is selected from HCoV-229E, HCoV-NL63, HCoV-OC43, HCoV-HKU1, MERS-CoV, SARS-CoV-1 and SARS-CoV-2, preferably from MERS-CoV, SARS-CoV-1 and SARS-CoV-2.

The present invention also concerns a pharmaceutical composition comprising at least one compound for use of the invention, as described hereinabove, and at least one pharmaceutically acceptable carrier, for use in the treatment and/or prevention of viral infections as described hereinabove.

According to one embodiment, the pharmaceutical composition for use of the invention comprises, in addition to the at least one compound for use of the invention, at least one additional active ingredient, *e.g*., an active ingredient selected from antiviral agents; neuraminidase inhibitors; M2 proton channel blockers; anti-interleukins 6; JAK inhibitors; interferons; a macrolide, preferably selected from the group consisting of azithromycin, clarithromycin, erythromycin, spiramycin, telithromycin; another active ingredient selected from BXT-25, chloroquine, hydroxychloroquine, brilacidin, dehydroandrographolide succinate, APN01, fingolimod, methylprednisolone, thalidomide, bevacizumab, sildenafil citrate, carrimycin, nicotine, and mixtures thereof; as described hereinabove.

In a preferred embodiment, the pharmaceutical composition for use of the invention comprises, in addition to the at least one compound for use of the invention, at least one additional active ingredient selected amongst an antiviral agent, a neuraminidase inhibitor such as oseltamivir, zanamivir, peramivir or laninamivir; a M2 proton channel blocker such as adamantadine or remantanide; an anti-interleukin 6 such as tocilizumab, siltuximab, sarilumab, sirukumab, clazakizumab or olokizumab; a JAK inhibitor, such as barcitinib, fedratinib or ruxolitinib; an interferon such as interferon beta-1a (IFN-β-1a), interferon beta-1b (IFN-β-1b) or peginterferon beta-la; macrolides selected from the group comprising azithromycin, clarithromycin, erythromycin, spiramycin and telithromycin; another active ingredient selected from BXT-25, chloroquine, hydroxychloroquine, brilacidin, dehydroandrographolide succinate, APN01, fingolimod, methylprednisolone, thalidomide, bevacizumab, sildenafil citrate, carrimycin, nicotine, and a mixture thereof.

The compounds of the invention may be used in monotherapy or in combination therapy in a subject in need of therapeutic and/or preventive treatment. Thus, according to a first embodiment, the compound for use of the invention is administered to the subject without any other active ingredient. Thus, according to a second embodiment, the compound for use of the invention is administered to the subject in combination with at least one additional active ingredient, e.g., an active ingredient selected from antiviral agents; neuraminidase inhibitors; M2 proton channel blockers; anti-interleukins 6; JAK inhibitors; interferons; macrolides selected from the group comprising azithromycin, clarithromycin, erythromycin, spiramycin and telithromycin; another active ingredient selected from BXT-25, chloroquine, hydroxychloroquine, brilacidin, dehydroandrographolide succinate, APN01, fingolimod, methylprednisolone, thalidomide, bevacizumab, sildenafil citrate, carrimycin, nicotine, and mixtures thereof; as described hereinabove

In one embodiment, the compound is administrated to the subject sequentially, simultaneously and/or separately with the other active ingredient as described hereinabove.

According to one embodiment, the subject in need of therapeutic and/or preventive treatment is diagnosed by a health professional. In practice, viral infections are diagnosed by any examination routinely carried out in the medical setting, including direct diagnosis, *i.e.* identification of the virus or its constituents, for example from a respiratory specimen, or indirect diagnosis, *i.e.* the detection of antibodies specific to the infection.

COVID-19 severity may be assessed according to the World Health Organization (WHO) criteria of severity as follows:
- mild: cases showing mild clinical symptoms with no sign of pneumonia on imaging.
- moderate: cases showing fever and respiratory symptoms (such as a cough, shortness of breath, and/or chest tightness) with radiological findings of pneumonia and requiring (O₂): 3L/min < oxygen < 5L/min
- severe: cases meeting any of the following criteria:
   - respiratory distress (respiratory rate (RR) ≧ 30 breaths/ min);
   - oxygen saturation (SpO₂) ≤ 93% at rest in ambient air; or SpO₂ ≤ 97% with O₂ > 5L/min;
   - ratio of artery partial pressure of oxygen/inspired oxygen fraction (PaO₂/FiO₂ ≦ 300 mmHg (1 mmHg = 0.133 kPa), PaO₂/FiO₂ in high-altitude areas (at an altitude of over 1,000 meters above the sea level) shall be corrected by the following formula: PaO₂/FiO₂ [multiplied by] [Atmospheric pressure (mmHg)/760]; and/or
   - chest imaging that showed obvious lesion progression within 24-48 hours > 50%.
- critical: cases meeting any of the following criteria:
   - respiratory failure and requiring mechanical ventilation;
   - shock; and/or
   - multiple organ failure (extra pulmonary organ failure) requiring admission to intensive care unit (ICU).

According to one embodiment, the subject suffers from mild COVID-19, moderate COVID-19, severe COVID-19 or critical COVID-19. According to one embodiment, the subject suffers from mild-to-moderate COVID-19. According to one embodiment, the subject suffers from severe-to-critical COVID-19.

According to one embodiment, the subject, especially the subject suffering from mild-to-moderate COVID-19 or from severe-to-critical COVID-19, is not hospitalized. According to one embodiment, the subject, especially the subject suffering from mild-to-moderate COVID-19 or from severe-to-critical COVID-19, is hospitalized. In one embodiment, the subject is hospitalized but does not require admission to intensive care unit (ICU). In one embodiment, the subject is hospitalized and requires admission to ICU.

According to one embodiment, the subject, especially the subject suffering from mild-to-moderate COVID-19 or from severe-to-critical COVID-19, requires oxygen therapy. In one embodiment, the subject requires non-invasive ventilation (NIV).

Severe-to-critical COVID-19 may alternatively be defined as COVID-19 requiring hospitalization and either NIV or high flow oxygen therapy, instead of being assessed according to the WHO as described hereinabove.

Preferably, the subject in need of therapeutic and/or preventive treatment is a warm-blooded animal, more preferably a human. According to one embodiment, the subject is a male. According to one embodiment, the subject is a female.

In the invention, the subject may be of any age. According to one embodiment, the subject is an adult, *i.e.* over 18 years of age. According to one embodiment, the subject is a child, *i.e.* under 18 years of age. According to one embodiment, the subject is an infant, *i.e.* having an age of more than one month and less than two years. According to one embodiment, the subject is a newborn, *i.e.* having an age from birth to less than one month.

According to one embodiment, the subject does not suffer from any underlying pathology.

According to one embodiment, the subject is at risk of developing a disease caused by a viral infection. According to one embodiment, the subject is at risk of developing a disease caused by a respiratory infection, such as influenza A and/or influenza B. According to one embodiment, the subject is suffering from influenza A and/or influenza B and is at risk of developing a respiratory or an extra-respiratory complication.

According to one embodiment, the subject is at risk of developing a disease caused by a coronavirus infection. According to one embodiment, the subject is at risk of developing a disease caused by SARS-CoV-2 infection, such as COVID-19. According to one embodiment, the subject suffering from COVID-19 is at risk of developing a respiratory or an extra-respiratory complication as described above.

According to one embodiment, the subject is suffering from at least one risk factor *i.e.* a preexisting disease, condition, habit or behavior that may lead to an increased risk of developing a severe or critical form of the disease caused by a coronavirus infection as described above.

According to one embodiment, the subject is an individual of any age with certain chronic conditions, such as HIV/AIDS, asthma, or chronic heart or lung disease. According to one embodiment, the subject is an adult with chronic cardiac and/or respiratory pathology. According to one embodiment, the subject is a pregnant woman. According to one embodiment, the subject is an elderly individual. According to one embodiment, the subject is an obese person (BMI>35). According to one embodiment, the subject is profoundly immunocompromised.

This invention also relates to the use of a compound as described hereinabove in the treatment and/or prevention of viral infections as described hereinabove.

This invention also relates to the use of a compound as described hereinabove in the manufacture of a medicament for the treatment and/or prevention of viral infections as described hereinabove.

This invention also relates to a method for the treatment and/or prevention of viral infections as described hereinabove in a subject in need thereof, comprising a step of administrating to said subject a therapeutically effective amount of a compound as described hereinabove.

Another object of the invention is a kit-of-parts comprising a first part comprising a compound of the invention as described hereinabove, and a second part comprising another active ingredient selected from antiviral agents; neuraminidase inhibitors; M2 proton channel blockers; anti-interleukins 6; JAK inhibitors; interferons; macrolides selected from the group comprising azithromycin, clarithromycin, erythromycin, spiramycin and telithromycin ; another active ingredient selected from BXT-25, chloroquine, hydroxychloroquine, brilacidin, dehydroandrographolide succinate, APN01, fingolimod, methylprednisolone, thalidomide, bevacizumab, sildenafil citrate, carrimycin, nicotine, and mixtures thereof; as described hereinabove.

In a preferred embodiment, the kit-of-parts of the invention comprises a first part comprising a compound of the invention as described hereinabove, and a second part comprising another active ingredient selected from an antiviral agent, a neuraminidase inhibitor such as oseltamivir, zanamivir, peramivir or laninamivir; a M2 proton channel blocker such as adamantadine or remantanide; an anti-interleukin 6 such as tocilizumab, siltuximab, sarilumab, sirukumab, clazakizumab or olokizumab; a JAK inhibitor, such as barcitinib, fedratinib or ruxolitinib; an interferon such as interferon beta-1a (IFN-β-1a), interferon beta-1b (IFN-β-1b) or peginterferon beta-la; another active ingredient selected from BXT-25, chloroquine, hydroxychloroquine, brilacidin, dehydroandrographolide succinate, APN01, fingolimod, methylprednisolone, thalidomide, bevacizumab, sildenafil citrate, carrimycin, nicotine, and macrolides selected from the group comprising azithromycin, clarithromycin, erythromycin, spiramycin and telithromycin, and a mixture thereof.

In one embodiment, the kit-of-parts of the invention comprises a first part comprising compound of the invention, or a pharmaceutically acceptable salt or solvate thereof or prodrug thereof, and a second part comprising another active ingredient such as oseltamivir or azythromycin.

### Methods of administration

The compounds of the invention as describes hereinabove, may be **administered** by oral, parenteral (e.g., intramuscular, intraperitoneal, intravenous, ICV, intracisternal injection or infusion, subcutaneous injection, or implant), by inhalation spray, nasal, rectal, sublingual, or topical routes of administration and may be formulated, alone or together, in suitable dosage unit formulations containing conventional non-toxic pharmaceutically acceptable carriers, adjuvants and vehicles appropriate for each route of administration. In addition to the treatment of warm-blooded animals, such as mice, rats, horses, cattle, sheep, dogs, cats, monkeys, etc., the compounds of the invention are effective for use in humans. The pharmaceutical compositions for the administration of the compounds of this invention may conveniently be presented in dosage unit form and may be prepared by any of the methods well known in the art of pharmacy. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active object compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs.

Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material, such as glyceryl monostearate or glyceryl distearate may be employed. They may also be coated by the techniques described in the U.S. Patents 4,256,108; 4,166,452; and 4,265,874 to form osmotic therapeutic tablets for control release. Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

**Aqueous suspensions** contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol , such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin. Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents, such as those set forth above, and flavoring agents may be added to provide a palatable oral preparation. These compositions may be preserved by the addition of an anti-oxidant, such as ascorbic acid. Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

Syrups and elixirs may be formulated with sweetening agents, for example glycerol, propylene glycol, sorbitol or sucrose. Such formulations may also contain a demulcent, a preservative and flavouring and colouring agents.

The pharmaceutical compositions may be in the form of a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. In addition, fatty acids, such as oleic acid find use in the preparation of injectables. The compounds of the present invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols. For topical use, creams, ointments, jellies, solutions or suspensions, etc., containing the compounds of the present invention are employed. (For purposes of this application, topical application shall include mouthwashes and gargles.)

In the treatment or prevention of viral infections, an appropriate dosage level will generally be about 0.01 to 500 mg per kg patient body weight per day which can be administered in single or multiple doses. Preferably, the dosage level will be about 0.1 to about 350 mg/kg per day; more preferably about 0.5 to about 100 mg/kg per day. A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the compositions are preferably provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, and 1000.0 milligrams of the active ingredient for the symptomatic adjustment of the dosage to the patient to be treated. For example the dosage may comprise from 100mg/day to 5000mg/day, preferably from 500mg/day to 1000mg/day. The compounds may be administered on a regimen of 1 to 4 times per day, preferably once, twice or three times per day. Three times per day has been fond suitable. The duration of the treatment will depend from the patent and is determined by the physician. It can be from one day to one year or even longer, preferably from one week to three months, more preferably from two weeks to six weeks. It will be understood, however, that the specific dose level and frequency of dosage and duration for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

### BRIEF DESCRIPTION OF THE DRAWINGS

**Figure 1** is a histogram showing the survival rate of mice during the fourteen days of experiment.
**Figure 2** is a histogram showing the weight (Fig. 2A) and weight loss (Fig. 2B) of mice from D0 to D14.
**Figure 3** is a histogram showing the effect of treatments on clinical parameters from D0 to D14.
**Figure 4** is a histogram showing the viral load in lung of infected mice 8 days after the infection with 500 PFU of influenza A virus.

### EXAMPLES

The present invention is further illustrated by the following examples.

### Example 1: Synthesis of compounds of the invention

### Materials and Methods

All materials were obtained from commercial suppliers and used without further purification. Thin-layer chromatography was performed on TLC plastic sheets of silica gel 60F254 (layer thickness 0.2 mm) from Merck. Column chromatography purification was carried out on silica gel 60 (70-230 mesh ASTM, Merck). Melting points were determined either on a digital melting point apparatus (Electrothermal IA 8103) and are uncorrected or on a Kofler bench type WME (Wagner & Munz). IR, ¹H, ¹⁹F and ¹³C NMR spectra confirmed the structures of all compounds. IR spectra were recorded on a Perkin Elmer Spectrum 100 FT-IR spectrometer and NMR spectra were recorded, using CDCl₃, CD₃CN, D₂O or DMSO-d₆ as solvent, on a Bruker AC 300, Advance DRX 400 and Advance DRX 500 spectrometers, for ¹H, 75 or 100 MHz for ¹³C and 282 or 377 MHz for ¹⁹F spectra. Chemical shifts (δ) were expressed in parts per million relative to the signal indirectly (i) to CHCl₃ (δ 7.27) for ¹H and (ii) to CDCl₃ (δ 77.2) for ¹³C and directly (iii) to CFCl₃ (internal standard) (δ 0) for ¹⁹F. Chemical shifts are given in ppm and peak multiplicities are designated as follows: s, singlet; br s, broad singlet; d, doublet; dd, doublet of doublet; t, triplet; q, quadruplet; quint, quintuplet; m, multiplet. The high-resolution mass spectra (HRMS) were obtained from the "Service central d'analyse de Solaize" (Centre national de la recherche scientifique) and were recorded on a Waters spectrometer using electrospray-TOF ionization (ESI-TOF).

### General experimental procedures

### Step 1: Synthesis of the compound of formula A-1

The compound of formula D (1.0 equiv.) is dissolved in dichloromethane. Nicotinamide of formula E (1.50 equiv.) and TMSOTf (1.55 equiv.) are added at room temperature. The reaction mixture is heated under reflux and stirred until the reaction is complete. The mixture is cooled to room temperature and filtered. The filtrate is concentrated to dryness to give tetraacetate A-1.

### Step 2: Synthesis of the compound of formula A-2

Tetraacetate A-1 is dissolved in methanol and cooled to -10 °C. Ammonia 4,6 M in methanol (3,0 equivalents) at -10 °C is added and the mixture is stirred at this temperature until the reaction is complete. Dowex HCR (H+) resin is added up to pH 6-7. The reaction mixture is heated to 0 °C and filtered. The resin is washed with a mixture of methanol and acetonitrile. The filtrate is concentrated to dryness. The residue is dissolved in the acetonitrile and concentrated to dryness. The residue is dissolved in the acetonitrile to give a solution of the compound of formula A-2.

### Step 3: Synthesis of the compound of formula A-3

The solution of the crude compound of formula A-2 in acetonitrile is diluted with trimethyl phosphate (10.0 equivalents). The acetonitrile is distilled under vacuum and the mixture is cooled to -10 °C. Phosphorus oxychloride (4,0 equivalents) is added at 10 °C and the mixture is stirred at 10 °C until the reaction is complete.

### Steps 4 and 5: Synthesis of the compound of formula I-A

The mixture obtained in step 3 above is hydrolyzed by the addition of a 50/50 mixture of acetonitrile and water, followed by the addition of methyl tert-butyl ether. The mixture is filtered and the solid is dissolved in water. The aqueous solution is neutralised by the addition of sodium bicarbonate and extracted with dichloromethane. The aqueous layer is concentrated to dryness to yield the crude formula I-A compound, which is purified on a DOWEX 50wx8 column with elution in water followed by a silica gel chromatographic column.

### Example 2: Evaluation of compounds of the invention on a murine Influenza virus pulmonary infection

The purpose of this study is to investigate the effects of an NMN derivative in the progression of a viral infection.

In order to directly assess the effects of compound **I-A** on lethal pulmonary viral infections, an in vivo study of severe pneumonia induced by the H1N1 influenza virus model PR/8/34 (protocol in progress) is undertaken. Mice are infected with a lethal dose (500 PFU/mouse) of the virus and are being treated or not with compound **I-A** and/or Tamiflu injected by IP route for 14 days. Characteristic clinical parameters (weight, temperature, state of alertness, response to stimulation, quality of respiration) of the animals' health status and mortality are monitored throughout the protocol. At D2 and D8 of infection, the progression of pneumonia (histology of the lung) is evaluated, multi-organ damage (histology of the heart, liver, kidneys, spleen) and pulmonary and systemic inflammatory and immune responses (immuno-phenotyping of pulmonary and blood leukocytes in broncoalveolar washings (BAL) and lymph nodes and determination of cytokines in plasma and BAL). A transcriptome analysis of all cell types presents in the lungs at D2 of the infection by the single cell RNAseq technique complete the study and allow to evaluate the effect of compound **I-A** on the expression of genes from different populations present in the lungs of animals with severe pneumonia.

### I. Materials and Methods

### Material

### Animals:

108 Balb/c male mice, 7-week-old at the arrival are obtained from Janvier Labs, Le Genest St Isle, 53941 St Berthevin, France. Each animal is identified by a unique animal number written on the tail/cage when assigned to the groups. Each cage is numbered. Based on the animal number / cage and number of cage, the animals are assigned of unique number with the name of group and mouse number.

The matching cards that are used to identify cages where experimental animals are housed will contain the following information: the name of the experiment, the number of the experiment and the cage number.

### Compound:

The NMN derivative is manufactured according to Example 1 or commercially purchased and stored at +4°C until use. Vehicle is physiological buffer.

### Methods

### 1. Preparation of formulation:

The powder of compound **I-A** is dissolved in vehicle (the solution is used at room temperature for maximum 1 day) at 6 mg/ml. A fresh sample for each administration is prepared every day except the week end (the solution is prepared on Saturday and is used on saturday and sunday).

The product is administered intraperitoneally once daily for 15 days.

Mice are weighed daily to adapt the volume of compound to be administred.

### 2. Influenza virus H1N1 PR/8/34 strain preparation

At Day 0, mice are infected with a lethal dose (500 PFU/mouse) of Influenza virus H1N1 PR/8/34 by intranasal route.

### 3. Experimental groups

Group description:
Group 1: Vehicle (i.p.)
Group 2: Tamiflu 1mg/kg (subactive dose)
Group 3: Test compound 185mg/kg (compound **I-A**)
Group 4: Tamiflu 1mg/kg + test compound 185 mg/kg

Group repartition:
For each group
- 12 mice for survival
- 5 mice for lung/lymph nodes collection and treatment for cytometry at day 2; blood collection
- 5 mice for lung/lymph nodes collection and treatment for cytometry at day 8; blood collection
- 5 mice for lung collection and preparing for single cell RNA sequencing at day 2

Each group will involve 27 mice.

As set forth in the regulations for Non-clinical Laboratory Studies, test and control animal groups is maintained under identical conditions. The duration of study is 15 days.

At D0, mice were infected with 500 PFU/mouse of Influenza virus H1N1 PR/8/34 by intranasal route.

Mice were i.p treated during all the experiment (D0 to D14) once per day. Last injection occurred 24 hours before sacrifice.

At D2, from 5 mice of each group, blood, lymph nodes and lungs are collected. Blood is collected by retro-orbital sampling. 50µL is immediately added to 200µL of percloric acid 0,1N and thorough mixing is achieved by inverting the tube several times. Another aliquot (300uL minimum) are sampled for cytometry analysis. The rest of blood is centrifuged, the plasma is collected and frozen until potential shipment to the sponsor.

Lungs was also collected from 5 other mice for treatment for RNA sequencing.

At D8, from 5 mice of each group, blood, lymph nodes and lungs were collected. Blood is collected by retro-orbital sampling. An aliquot (300uL minimum) is sampled for cytometry analysis. The rest of blood is centrifuged, the plasma is collected and frozen until potential shipment to the sponsor.

The weight loss, the health scores and the mortality are assessed for 15 days (D0 to D14).

At D14, lungs from remaining mice are collected.

### 4. Dosing

The vehicle (physiological buffer) is administered once a day by intraperitoneal route.

The Tamiflu reference is administrated twice a day at 1 mg/kg for group 2 and 4 by oral gavage.

Compound **I-A** is administrated once a day at 185 mg/kg by intraperitoneal route.

### 5. Infection

Mice are flash anesthetized with isoflurane and then are intranasally infected with the Influenza virus H1N1 PR/8/34 at 500 PFU/mouse.

### 6. Body weight, survival rate and clinical examination

The mortality, body weight and clinical signs are recorded every day until the end of the experiment (D14) for 12 mice per groups.

The clinical score is established as followed:
1: healthy mouse
2: mouse showing signs of malaise, including slight piloerection, slightly changed gait and increased ambulation
3: mouse showing signs of strong piloerection, constricted abdomen, changed gait, periods of inactivity
4: mouse with enhanced characteristics of the previous group, but showing little activity and becoming moribund
5: dead mouse

The animal appearance and behavior are assessed at least daily from the start and until the end of the experimental phase. Any abnormal findings are recorded in the raw data. Body weight measurement and clinical examination are also carried out before the animals are split into groups.

### 7. Blood collection at day 2 and day 8

Retro-orbital blood collection is performed 48 hours and 8 days after the infection with EDTA anticoagulant.

At day 2, 50 µL of blood is mixed with 200 µL of perchloric acid (0,5N) by inverting the tube several times. Samples are stored at -70°C until shipment to sponsor.

At day 2 and 8, a minimum of 300µL of blood is used for cytometry analysis.

The rest of blood is centrifuged to collect plasma and stored at -70°C until shipment.

### 8. Cell isolation from lungs and draining lymph nodes

At day 2 and 8, lungs and draining lymph nodes are collected in cold RPMI + 10% FCS.

Lungs is cut in small pieces and then digested in collagenase solution at 37°C for 30 minutes. Cell suspension is then filtered on 40 µm cell strainer.

Draining lymph nodes is smashed on a 40 µm cell strainer on top of a 50mL tube with a syringe piston.

Cell suspension is centrifuged at 400g during 5 minutes at 4°C. The pellet is resuspended in 1-5mL of cold RPMI + 10% FCS.

Viable cells are then counted and used for cytometry.

### 9. Cytometry analysis on whole blood, lung and draining lymph nodes

Whole blood and cells isolated from lungs and draining lymph nodes are labelled with antibodies.

For lung cells, 4 panels are performed:
Panel 1: identification of alveolar macrophages
Panel 2: identification of T cell subsets and NK cells
Panel 3: identification of interstitial macrophages and recruited monocytes
Panel 4: identification of dendritic cell subsets and plasmacytoid cells

For draining lymph node cells, 3 panels are performed:
Panel 1: identification of T cell subsets and NK cells
Panel 2: identification of dendritic cell subsets
Panel 3: identification of macrophages and plasmacytoid dendritic cells

For whole blood, 3 panels are performed:
Panel 1: identification of TCR αβ⁺ T cell subsets, NK cells and B cells.
Panel 2: identification of TCRγδ⁺T cells and regulatory T cells.
Panel 3: identification of monocytes, dendritic cells and plasmacytoid dendritic cell.

### II. Results and discussion

### 1. Survival rate

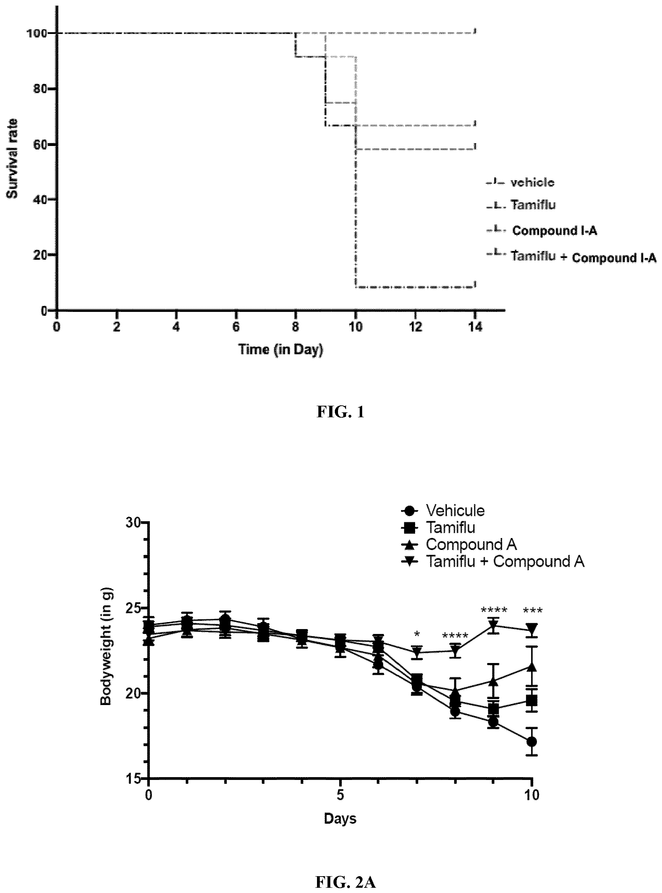

**Figure 1** shows the survival rate of mice during the fourteen days of experiment.

It was observed that compared to untreated control animals, compound **I-A (NMN)** allows to reduce animal mortality.

As shown in **Figure 1****,** 92 % of mortality was obtained for mice treated with the vehicle at D14.

Compared to untreated control animals, it was observed that treatments with oseltamivir (Tamiflu®) at 1mg/kg/day or compound **I-A,** improved greatly mice survival, respectively with a survival rate of 58 % and 67% at D14.

Interestingly, compound **I-A** appears to be more effective than oseltamivir (1mg/kg/day).

Remarkably, co-administration of compound **I-A** and oseltamivir (2mg/kg/day) protects animals from lethality induced by H1N1 infection with a survival rate of 100% at D14.

### 2. Body weight

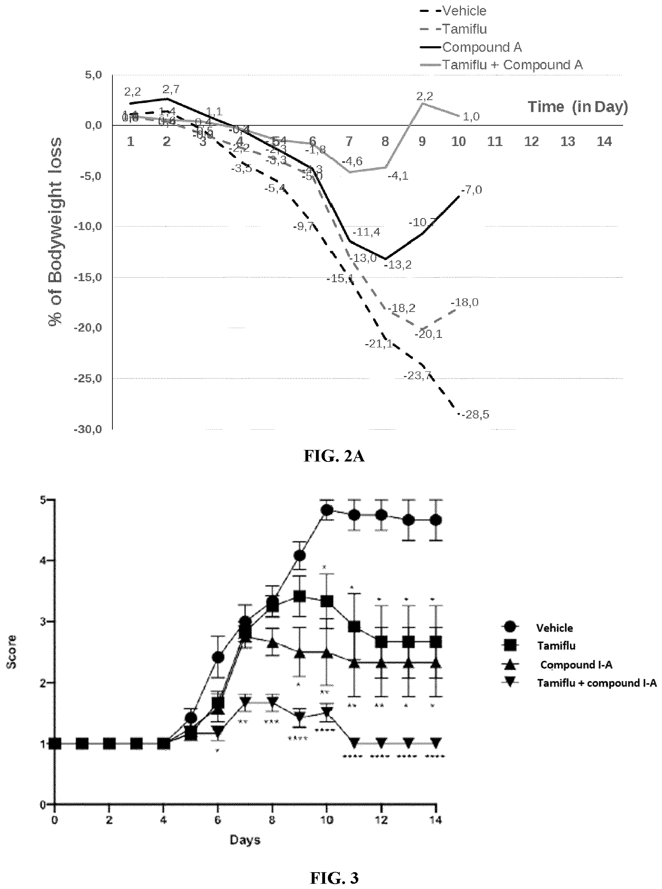

**Figure 2** shows the body weight evolution of mice from D0 to D14.

As shown in **figure 2**, from D4 to D9, the mice from vehicle group showed 23 % of bodyweight loss on average.

Daily treatments with compound **I-A** or oseltamivir alone allows a slight improvement of this parameter in comparison to vehicle, whereas the combination oseltamivir + compound **I-A** has significantly contributed to maintained body weight of infected mice.

### 3. Clinical score

**Figure 3** shows the effect of treatments on clinical parameters from D0 to D14, as follow:
1: healthy mouse
2: mouse showing signs of malaise, including slight piloerection, slighlty changed gait and increased ambulation
3: mouse showing signs of strong piloerection, constricted abdomen, changed gait, periods of inactivity
4: mouse with enhanced characteristics of the previous group, but showing little activity and becoming moribund
5: dead mouse

As shown Mice treated with vehicle showed signs of malaise, slight piloerection and increased ambulation 5 days following the infection. Strong piloerection appeared on Day 7 with long periods of inactivity.

Treatments with oseltamivir or compound **I-A** significantly improved clinical states of the mice.

The combination has limited strong clinical signs and all the mice of this group were recovered on Day 11.

### 4. Viral load

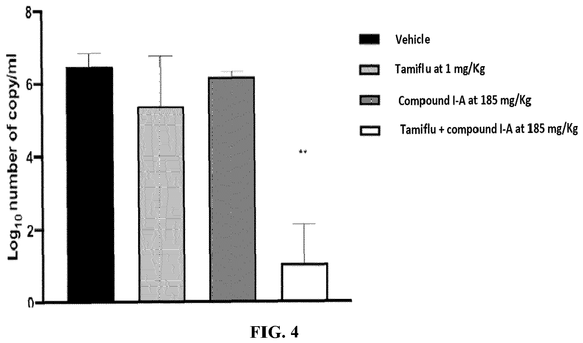

**Figure 4** shows the viral load in lung of infected mice at D8.

As shown in **figure 4** the treatment with compound **I-A** and oseltamivir had no impact on viral load in lung, 8 days after the infection with 500 PFU of influenza A virus.

However, combination of compound **I-A** with oseltamivir had significantly reduced the quantity of H1N1 particles in comparison to the vehicle group.

### III. Conclusion

This study on H1N1 infection already highlights the efficacy of treatment with compound **I-A** to prevent mortality during severe pneumonia and the superiority of the association between an antiviral molecule and compound **I-A** compared to treatment with monotherapies of either the antiviral or compound **I-A.**

This study on H1N1 infection allows also to unequivocally determine the effects of treatment on the progression of the pathology and the onset of acute respiratory disease, focusing on innate and acquired pulmonary and systemic (heart, spleen, liver and kidney) immune responses as well as cytokine storm.

### Example 3: Evaluation of compounds of the invention on a golden Syrian hamster SARS-CoV-2 infection

The purpose of this study is to investigate the effects of an NMN derivative in the progression of a coronavirus infection, in particular a SARS-CoV-2 infection.

### Materials and Methods

### Material

### Animals:

120 Golden syrian hamsters (6 to 10 weeks old) are obtained for the experiment. Each animal is identified by a unique animal number written on the tail/cage when assigned to the groups. Each cage is numbered. Based on the animal number / cage and number of cages, the animals are assigned of unique number with the name of group and hamster number.

The matching cards that are used to identify cages where experimental animals are housed will contain the following information: the name of the experiment, the number of the experiment and the cage number.

### Compound:

The NMN derivative is manufactured according to Example 1 or commercially purchased and stored at +4°C until use. Vehicle is physiological buffer.

### Methods

### 10. Preparation of formulation:

The powder of compound **I-A** is dissolved in vehicle (the solution is used at room temperature for maximum 1 day). A fresh sample for each administration is prepared every day except the week-end (the solution is prepared on Saturday and is used on Saturday and Sunday).

The product is administered intraperitoneally once daily for 15 days.

Hamster are weighed every day to adapt the volume of compound to be administered.

### 1. SARS-CoV-2 strain preparation

The animals are kept in Biosafety Level-2 housing and given access to standard pellet feed and water ad libitum until virus challenge in our Biosafety Level-3 animal facility. Phosphate-buffered saline (PBS) is used to dilute virus stocks to the desired concentration, and inocula are back-titrated to verify the dose given. Dulbecco's Modified Eagle Medium (DMEM) containing 10⁵ plaque-forming units in 100µl of SARS-CoV-2 is intranasally inoculated at day 0.

### 2. Experimental groups

6 experimental groups are investigated. Compound **I-A** (114 mg/Kg/day), compound **I-E** (114 mg/Kg/day), anti-viral (Remdesivir (17 mg/Kg/day) ou hydroxychloroquine (91 mg/Kg/day)) or a combination of compound **I-A** or compound **I-E** and Remdesivir or hydroxychloroquine is evaluated for efficacy 20 animal per group to follow the evolution of the pathology under each treatment. The following parameters are investigated:
- Viral load
- Histopathology of the organs
- Immunohistology to analyse macrophages differenciation, immune cells infiltrations in the selected tissues
- qPCR of the genes regulated by the virus (IFN-gamma, IL-4, IL-6, IL-10, IL-13, TNF-alpha, IL-21, TGFbeta1, CCL17, CCL22, CCR4, FOXP3, IL-12p40, gamma-actin)
- Circulating cytokines levels (IL-6, IL-10, IFN, TNF-alpha, MIP-1A, MCP-1, IP-10, TGF-beta1)
- NAD/NADH ratios in blood and tissues samples

These analyses are performed on 5 animals of each group at day 2, day 4, day 7 and day 14 post-infection. Read-outs are performed on blood, lungs, spleen, kidneys, liver and intestines.

### Example 4: Evaluation of compounds of the invention on SARS-CoV-2 infected human epithelial cells and on human immune cell activated by SARS-CoV-2 infected human epithelial cells

The purpose of this study is to evaluate the effects of an NMN derivative, especially compound **I-A,** on the activation and function of coronavirus-infected human lung epithelial cells and macrophages and human dendritic cells in contact with infected cells.

Primary type II alveolar epithelial cells cultured in the presence or absence of compound **I-A** alone or in combination with antiviral treatment (remdesivir or hydroxychloroquine) will be infected with the virus (SARS-CoV-2 or another human pathogenic coronavirus strain).

The effect of compound **I-A** is then evaluated on viral replication and infection as well as activation and release of cytokines by alveolar cells. Gene expression levels (RNAseq) in infected alveolar cells under the different treatment conditions are also analyzed.

At the same time, human circulating monocytes are differentiated into macrophages and dendritic cells that may or may not be treated with compound **I-A** alone or in combination with antiviral treatment (remdesivir or hydroxychloroquine). These immune cells are then brought into contact with coronavirus-infected epithelial cells and their levels of infection, their phagocytosis, cytokine secretion and activation (immunophenotyping) capacities are determined as well as the analysis of their differential gene expressions (RNA seq).

### Example 5: A prospective, multicentric, randomised, placebo-controlled double-blind study in COVID-19 patients

The overall objective of the study is to determine the therapeutic effect and tolerance of compound I-A in 150 patients with moderate, severe pneumonia associated with Coronavirus disease 2019 (COVID-19). The study has multiple Randomized Placebo-Controlled Trials (cmRCT) design. Compound I-A is administered to consenting adult patients hospitalized with COVID-19 either diagnosed with moderate or severe pneumonia.

The conditions are as follows:
Treatment regimen : 3 x 250mg / day of **compound I-A** vs. Placebo (on top of standard of care) for 28 days

### Inclusion Criteria:

- 18 years or older
- Hospitalized patient
- Has laboratory-confirmed SARS-CoV-2 infection as determined by PCR, or other commercial or public health assay ≤ 4 days before randomization
- Moderate to severe COVID-19 associated disease
- For moderate patients : peripheral capillary oxygen saturation (SpO₂) > 94% on room air at screening and radiographic evidence of pulmonary infiltrates
- For severe patients: Peripheral capillary oxygen saturation (SpO₂) ≤ 94% or requiring supplemental oxygen at screening
- Willing and able to provide written informed consent prior to performing study procedures

### Exclusion Criteria:

- Participation in any other clinical trial of an experimental treatment for COVID-19
- Concurrent treatment with other agents with actual or possible direct acting antiviral activity against SARS-CoV-2 is prohibited < 24 hours prior to study medication initiation
- SOFA >10
- Stage 4 severe chronic kidney disease or requiring dialysis (i.e. eGFR < 30)
- Pregnant women or women who are breastfeeding
- Immunocompromised patients taking medication upon screening
- Consideration by the investigator, for any reason, that the subject is an unsuitable candidate to receive study treatment

### Outcome Measures

### Primary Outcome Measures :

### Percentage of subjects reporting each severity rating on a 7-point ordinal scale [Time Frame: Day 7]

*a. Not hospitalized, no limitations on activities*
*b. Not hospitalized, limitation on activities;*
*c. Hospitalized, not requiring supplemental oxygen;*
*d. Hospitalized, requiring supplemental oxygen;*
*e. Hospitalized, on non-invasive ventilation or high flow oxygen devices;*
*f. Hospitalized, on invasive mechanical ventilation or ECMO;*
g. *Death.*

### Secondary Outcome Measures :

1. Overall Survival [Time Frame: 7, 14, 28 days]
2. 7-day, 14-day and 28-day ventilator free-days [Time Frame: 28 days]
3. PaO₂/FiO₂ ratio [Time Frame: day 1 to day 14]
   evolution of PaO2/FiO2 ratio
4. time to oxygen supply independency [Time Frame: 7-day, 14-day and 28-day] time to oxygen supply independency
5. duration of hospitalization [Time Frame: 7-day, 14-day and 28-day] duration of hospitalization
6. time to negative viral excretion [Time Frame: 7-day, 14-day and 28-day] time to negative viral excretion
7. time to ICU discharge [Time Frame: 7-day, 14-day and 28-day] time to ICU discharge
8. time to hospital discharge [Time Frame: 7-day, 14-day and 28-day] time to hospital discharge

### Labs/biomarkers at 7-day, 14-day and 28-day:

- *NFS, VS, PCR, Fibrinogene*
- *NAD*
- *Lymphocyte subpopulation (CD8, CD4, CD16, CD56) eurofins*
- *CD57 NK*
- *CD19 Lympho B*
- *IL-1α and IL_1β, interleukin-2,interleukin-4, interleukin-6, interleukin-8,interleukin-10, vascular endothelium growth factor (VEGF), interferon y, epidermal growth factor (EGF), monocyte chemoattractant protein type 1 (MCP-1) and TNFα.*
- *C3, C4, CH50*
- *DDID, A T3, TCK, TP, Proteins S and C*
- *CPK, CPKMB, Troponin, BNP, myoglobin, procalcitonin*
- *Transaminases, PAL, GGT, LDH, bilirubin, calcium levels*
- *Complete ionogram (Na,K,Cl,Ra,protidemie)*
- *Ferritinemie*
- *Lipase, Aldolase*

## Claims

1. Compound of Formula (I) 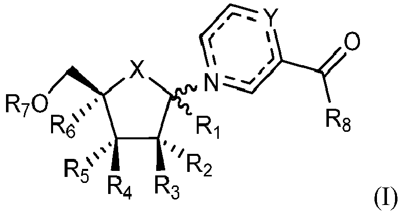 or a pharmaceutically acceptable salt or solvate thereof or prodrug thereof;
wherein:
X is selected from O, CH₂, S, Se, CHF, CF₂ et C=CH₂;
**R₁** is selected from H, azido, cyano, Ci-Cs alkyl, Ci-Csthio-alkyl, Ci-Cs heteroalkyl and OR; wherein R is selected from H and C₁-C₈ alkyl;
**R₂**, **R₃**, **R₄** et **R₅** are independently selected from H, halogen, azido, cyano, hydroxyl, C₁-C₁₂ alkyl, C₁-C₁₂ thioalkyl, C₁-C₁₂ heteroalkyl, C₁-C₁₂ haloalkyl and OR; wherein R is selected from H, C₁-C₁₂ alkyl, C(O)(C₁-C₁₂)alkyl, C(O)NH(C₁-C₁₂)alkyl, C(O)O(C₁-C₁₂)alkyl, C(O)aryl, C(O)(C₁-C₁₂)alkyl aryl, C(O)NH(C₁-C₁₂)alkyl aryl, C(O)O(C₁-C₁₂)alkyl aryl and C(O)CHR_{AA}NH₂; wherein R_{AA} is a side chain selected from a proteinogenic amino acid;
**R₆** is selected from H, azido, cyano, Ci-Cs alkyl, C₁-C₈ thio-alkyl, C₁-C₈ heteroalkyl and OR; wherein R is selected from H and C₁-C₈ alkyl;
**R₇** is selected from H, P(O)R₉R₁₀ et P(S)R₉R₁₀; wherein:
R₉ and R₁₀ are independently selected from OH, OR₁₁, NHR₁₃, NR₁₃R₁₄, C₁-C₈ alkyl, C₂-C₈ alkenyl, C₂-C₈ alkynyl, C₃-C₁₀ cycloalkyl, C₅-C₁₂ aryl, C₁-C₈ arylalkyl, C₁-C₈ alkylaryl, C₁-C₈ heteroalkyl, C₁-C₈ heterocycloalkyl, heteroaryl and NHCR_{α}R_{α'}C(O)R₁₂; wherein:
- R₁₁ is selected from C₁-C₁₀ alkyl, C₃-C₁₀ cycloalkyl, C₅-C₁₂ aryl, C₁-C₁₀ alkylaryl, substituted C₅-C₁₂ aryl, C₁-C₁₀ heteroalkyl, C₁-C₁₀ haloalkyl, - (CH₂)ₙC(O)(C₁-C₁₅)alkyl, -(CH₂)ₙOC(O)(C₁-C₁₅)alkyl, -(CH₂)ₙOC(O)O(C₁-C₁₅)alkyl, -(CH₂)ₙSC(O)(C₁-C₁₅)alkyl, -(CH₂)ₙC(O)O(C₁-C₁₅)alkyl and - (CH₂)ₙC(O)O(C₁-C₁₅)alkyl aryl; wherein n is an integer selected from 1 to 8; and P(O)(OH)OP(O)(OH)₂;
- R₁₂ is selected from hydrogen, C₁-C₁₀ alkyl, C₂-C₈ alkenyl, C₂-C₈ alkynyl, Ci-C₁₀ haloalkyl, C₃-C₁₀ cycloalkyl, C₃-C₁₀ cycloheteroalkyl, C₅-C₁₂ aryl, C₁-C₄ alkylaryl and C₅-C₁₂ heteroaryl; wherein said aryl or heteroaryl groups are optionally substituted by one or two groups selected from halogen, trifluoromethyl, C₁-C₆ alkyl, C₁-C₆ alkoxy and cyano;
- R₁₃ and R₁₄ are independently selected from H, C₁-C₈ alkyl and C₁-C₈ alkyl-aryl;
- R_{α} and R_{α'} are independently selected from an hydrogen, C₁-C₁₀ alkyl, C₂-C₁₀ alkenyl, C₂-C₁₀ alkynyl, C₃-C₁₀ cycloalkyl, C₁-C₁₀ thio-alkyl, C₁-C₁₀ hydroxylalkyl, C₁-C₁₀ alkylaryl and C₅-C₁₂ aryl, -(CH₂)₃NHC(=NH)NH₂, (1H-indol-3-yl)methyl, (1H-imidazol-4-yl)methyl and a side chain selected from a proteinogenic or non-proteinogenic amino acid; wherein said aryl groups are optionally substituted with a group selected from hydroxyl, C₁-C₁₀ alkyl, C₁-C₆ alkoxy, halogen, nitro and cyano;; or
R₉ and Rio together with the phosphorus atoms to which they are attached form a 6-membered ring wherein -R₉-R₁₀- represents -CH₂-CH₂-CHR-; wherein R is selected from hydrogen, C₅-C₆ aryl and C₅-C₆ heteroaryl, wherein said aryl or heteroaryl groups are optionally substituted by one or two groups selected from halogen, trifluoromethyl, C₁-C₆ alkyl, C₁-C₆ alkoxy and cyano; or
R9 and R10 together with the phosphorus atoms to which they are attached form a 6-membered ring wherein -R9-R10- represents -O-CH2-CH2-CHR-O-; wherein R is selected from hydrogen, C5-C6 aryl and C5-C6 heteroaryl, wherein said aryl or heteroaryl groups are optionally substituted by one or two groups selected from halogen, trifluoromethyl, C1-C6 alkyl, C1-C6 alkoxy and cyano
**R₈** is selected from H, OR, NHR₁₅, NR₁₅R₁₆, NH-NHR₁₃, SH, CN, N₃ and halogen;
wherein R₁₅ and R₁₆ are independently selected from H, C₁-C₈ alkyl and C₁-C₈ alkyl-aryl;
**Y** is selected from CH, CH₂, C(CH₃)₂ and CCH₃;
-̅-̅-̅ represents a single or double bond according to **Y**; and
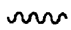 represents the alpha or beta anomer depending on the position of **R₁**,
or a compound of formula (Ia) 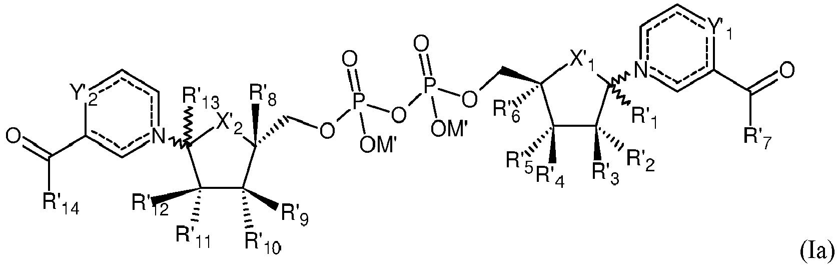 or pharmaceutically acceptable salts and/or solvates thereof or prodrugs thereof, wherein:
- X'₁ and X'₂ are independently selected from O, CH₂, S, Se, CHF, CF₂ and C=CH₂;
- R'₁ and R'₁₃ are independently selected from H, azido, cyano, C1-C8 alkyl, C1-C8 thio-alkyl, C1-C8 heteroalkyl and OR; wherein R is selected from H and C1-C8 alkyl;
- R'₂, R'₃, R'₄, R'₅, R'₉, R'₁₀, R'₁₁, R'₁₂ are independently selected from H, halogen, azido, cyano, hydroxyl, C1-C12 alkyl, C1-C12 thio-alkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl or C(O)CHR_{AA}NH₂, wherein R_{AA} is a side chain selected from a proteinogenic amino acid ;
- R'₆ and R'₈ are independently selected from H, azido, cyano, C1-C8 alkyl and OR; wherein R is selected from H and C1-C8 alkyl;
- R'₇ and R'₁₄ are independently selected from H, OR, NHR, NRR', NH-NHR, SH, CN, N₃ and halogen; wherein R and R' are each independently selected from H, C1-C8 alkyl, C1-C8 alkyl aryl;
- Y'₁ and Y'₂ are independently selected from CH, CH₂, C(CH₃)₂ or CCH₃;
- M' is selected from H or a suitable counterion;
- 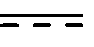 represents a single or a double bound depending on Y'₁ and Y'₂; and
- 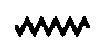 represents the alpha or beta anomer depending on the position of R'₁ and R'₁₃,
for use in the treatment and/or prevention of viral infections.

2. The compound for use according to claim **1**, wherein the viral infection is caused by at least one virus of the genus selected from *Influenzavirus, Coronavirus, Respirovirus, Pneumovirus, Metapneumovirus, Adenovirus, Enterovirus, Rhinovirus, Hepatovirus, Erbovirus, Aphtovirus, Norovirus, Alphavirus, Rubivirus, Flavivirus, Hepacivirus, Pestivirus, Ebola-like virus, Morbillivirus, Rubulavirus, Henipavirus, Arenavirus, Orthobunyavirus, Phlebovirus, Rotavirus, Simplexvirus, Varicellovirus* or *Cytomegalovirus,* preferably wherein the viral infection is a respiratory infection caused by at least one virus of the genus selected from *Influenzavirus, Coronavirus, Rhinovirus, Respirovirus, Pneumovirus* or *Metapneumovirus.*

3. The compound for use according to any one of claims **1** to **2**, wherein the viral infection is a respiratory infection caused by *Influenzavirus,* preferably influenza A or influenza B, preferably wherein the viral infection is a respiratory infection selected from H1N1, H3N2, H5N1, B/Yamagata/16/88-like and B/Victoria/2/87-like viruses.

4. The compound for use according to any one of claims **1** to **3**, wherein the viral infection is a coronavirus infection caused by a coronavirus selected from HCoV-229E, HCoV-NL63, HCoV-OC43, HCoV-HKU1, MERS-CoV, SARS-CoV-1 and SARS-CoV-2, preferably from MERS-CoV, SARS-CoV-1 and SARS-CoV-2, preferably wherein the viral infection is a SARS-CoV-2 infection causing coronavirus disease 2019 (COVID-19).

5. The compound for use according to any one of claims **1** to **4**, wherein the virus infection is a SARS-CoV-2 infection causing COVID-19 associated pneumonia or a SARS-CoV-2 infection causing COVID-19 associated acute respiratory distress syndrome (ARDS).

6. Compound of Formula (I) 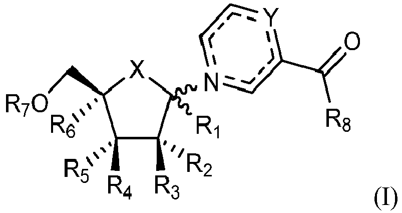 or a pharmaceutically acceptable salt or solvate thereof or prodrug thereof;
wherein:
**X** is selected from O, CH₂, S, Se, CHF, CF₂ et C=CH₂;
**R₁** is selected from H, azido, cyano, C₁-C₈ alkyl, C₁-C₈thio-alkyl, C₁-C₈ heteroalkyl and OR; wherein R is selected from H and C₁-C₈ alkyl;
**R₂, R₃, R₄** et **R₅** are independently selected from H, halogen, azido, cyano, hydroxyl, C₁-C₁₂ alkyl, C₁-C₁₂ thioalkyl, C₁-C₁₂ heteroalkyl, C₁-C₁₂ haloalkyl and OR; wherein R is selected from H, C₁-C₁₂ alkyl, C(O)(C₁-C₁₂)alkyl, C(O)NH(C₁-C₁₂)alkyl, C(O)O(C₁-C₁₂)alkyl, C(O)aryl, C(O)(C₁-C₁₂)alkyl aryl, C(O)NH(C₁-C₁₂)alkyl aryl, C(O)O(C₁-C₁₂)alkyl aryl and C(O)CHR_{AA}NH₂; wherein R_{AA} is a side chain selected from a proteinogenic amino acid;
**R₆** is selected from H, azido, cyano, Ci-Cs alkyl, C₁-C₈ thio-alkyl, C₁-C₈ heteroalkyl and OR; wherein R is selected from H and C₁-C₈ alkyl;
**R₇** is selected from H, P(O)R₉R₁₀ et P(S)R₉R₁₀; wherein:
R₉ and Rio are independently selected from OH, OR₁₁, NHR₁₃, NR₁₃R₁₄, C₁-C₈ alkyl, C₂-C₈ alkenyl, C₂-C₈ alkynyl, C₃-C₁₀ cycloalkyl, C₅-C₁₂ aryl, C₁-C₈ arylalkyl, C₁-C₈ alkylaryl, C₁-C₈ heteroalkyl, C₁-C₈ heterocycloalkyl, heteroaryl and NHCR_{α}R_{α'}C(O)R₁₂; wherein:
- R₁₁ is selected from C₁-C₁₀ alkyl, C₃-C₁₀ cycloalkyl, C₅-C₁₂ aryl, C₁-C₁₀ alkylaryl, substituted C₅-C₁₂ aryl, C₁-C₁₀ heteroalkyl, C₁-C₁₀ haloalkyl, - (CH₂)ₙC(O)(C₁-C₁₅)alkyl, -(CH₂)ₙOC(O)(C₁-C₁₅)alkyl, -(CH₂)ₙOC(O)O(C₁-C₁₅)alkyl, -(CH₂)ₙSC(O)(C₁-C₁₅)alkyl, -(CH₂)nC(O)O(C₁-C₁₅)alkyl and - (CH₂)ₙC(O)O(C₁-C₁₅)alkyl aryl; wherein n is an integer selected from 1 to 8; and P(O)(OH)OP(O)(OH)₂;
- R₁₂ is selected from hydrogen, C₁-C₁₀ alkyl, C₂-C₈ alkenyl, C₂-C₈ alkynyl, Ci-C₁₀ haloalkyl, C₃-C₁₀ cycloalkyl, C₃-C₁₀ cycloheteroalkyl, C₅-C₁₂ aryl, C₁-C₄ alkylaryl and C₅-C₁₂ heteroaryl; wherein said aryl or heteroaryl groups are optionally substituted by one or two groups selected from halogen, trifluoromethyl, C₁-C₆ alkyl, C₁-C₆ alkoxy and cyano;
- R₁₃ and R₁₄ are independently selected from H, C₁-C₈ alkyl and C₁-C₈ alkyl-aryl;
- R_{α} and R_{α'} are independently selected from an hydrogen, C₁-C₁₀ alkyl, C₂-C₁₀ alkenyl, C₂-C₁₀ alkynyl, C₃-C₁₀ cycloalkyl, C₁-C₁₀ thio-alkyl, C₁-C₁₀ hydroxylalkyl, C₁-C₁₀ alkylaryl and C₅-C₁₂ aryl, -(CH₂)₃NHC(=NH)NH₂, (1H-indol-3-yl)methyl, (1H-imidazol-4-yl)methyl and a side chain selected from a proteinogenic or non-proteinogenic amino acid; wherein said aryl groups are optionally substituted with a group selected from hydroxyl, C₁-C₁₀ alkyl, C₁-C₆ alkoxy, halogen, nitro and cyano;; or
R₉ and Rio together with the phosphorus atoms to which they are attached form a 6-membered ring wherein -R₉-R₁₀- represents -CH₂-CH₂-CHR-; wherein R is selected from hydrogen, C₅-C₆ aryl and C₅-C₆ heteroaryl, wherein said aryl or heteroaryl groups are optionally substituted by one or two groups selected from halogen, trifluoromethyl, C₁-C₆ alkyl, C₁-C₆ alkoxy and cyano;
**R₈** is selected from H, OR, NHR₁₅, NR₁₅R₁₆, NH-NHR₁₃, SH, CN, N₃ and halogen;
wherein R₁₅ and R₁₆ are independently selected from H, C₁-C₈ alkyl and C₁-C₈ alkyl-aryl;
**Y** is selected from CH, CH₂, C(CH₃)₂ and CCH₃;
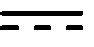 represents a single or double bond according to **Y**; and
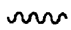 represents the alpha or beta anomer depending on the position of **R₁**,
or a compound of formula (Ia) 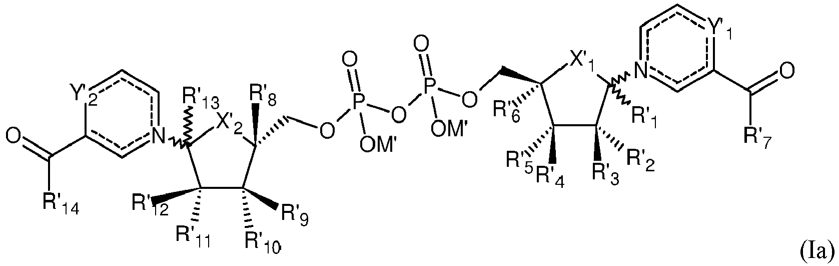
or pharmaceutically acceptable salts and/or solvates thereof or prodrugs thereof, wherein:
- X'₁ and X'₂ are independently selected from O, CH₂, S, Se, CHF, CF₂ and C=CH₂;
- R'₁ and R'₁₃ are independently selected from H, azido, cyano, C1-C8 alkyl, C1-C8 thio-alkyl, C1-C8 heteroalkyl and OR; wherein R is selected from H and C1-C8 alkyl;
- R'₂, R'₃, R'₄, R'₅, R'₉, R'₁₀, R'₁₁, R'₁₂ are independently selected from H, halogen, azido, cyano, hydroxyl, C1-C12 alkyl, C1-C12 thio-alkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl or C(O)CHR_{AA}NH₂, wherein R_{AA} is a side chain selected from a proteinogenic amino acid ;
- R'₆ and R'₈ are independently selected from H, azido, cyano, C1-C8 alkyl and OR; wherein R is selected from H and C1-C8 alkyl;
- R'₇ and R'₁₄ are independently selected from H, OR, NHR, NRR', NH-NHR, SH, CN, N₃ and halogen; wherein R and R' are each independently selected from H, C1-C8 alkyl, C1-C8 alkyl aryl;
- Y'₁ and Y'₂ are independently selected from CH, CH₂, C(CH₃)₂ or CCH₃;
- M' is selected from H or a suitable counterion;
- 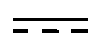 represents a single or a double bound depending on Y'₁ and Y'₂; and
- 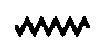 represents the alpha or beta anomer depending on the position of R'₁ and R'₁₃,
for use in the treatment and/or prevention of respiratory or extra-respiratory complications and/or infections of viral origin.

7. The compound for use according to claim **6**, for use in the treatment and/or prevention of pneumonia and/or acute respiratory diseases, acute respiratory distress syndrome (ARDS), acute respiratory failure.

8. The compound for use according to claim **6** or **7**, for use in the treatment and/or prevention of pneumonia and/or acute respiratory syndromes associated with COVID-19.

9. The compound for use according to any one of claims **1** to **8**, wherein **X** represents an oxygen.
- **X** represents an oxygen; and/or
- **R₁** and **R₆** each independently represents a hydrogen; and/or
- **R₂, R₃, R₄** and **R₅** each independently represents a hydrogen, or **R₂, R₃, R₄** and **R₅** each independently represents a OH; and/or
- **Y** represents a CH or a CH₂; and/or
- **R₇** represents P(O)R₉R₁₀, wherein R₉ and R₁₀ are as described in claim 1.

10. The compound for use according to any one of claims **1** to **9**, selected from:
| **Compounds (anomers)** | **Structure** |
|---|---|
| **I-A (beta)** | 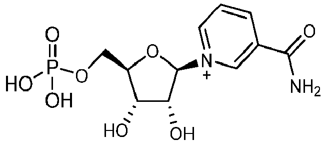 |
| **I-B (alpha)** | 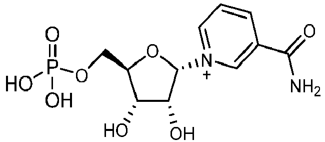 |
| **I-C (beta)** | 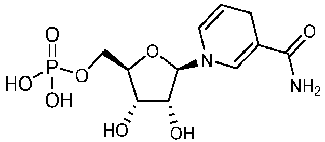 |
| **I-D (alpha)** | 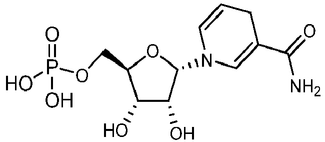 |
| **I-E (beta)** | 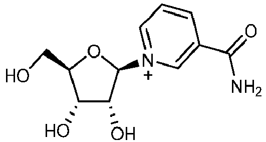 |
| **I-F (alpha)** | 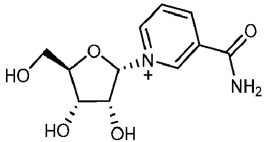 |
| **I-G (beta)** | 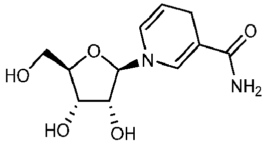 |
| **I-H (alpha)** | 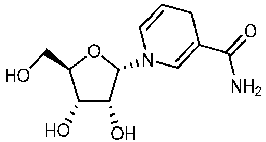 |
Or
| **Cpd n° (anomers)** | **Structure** |
|---|---|
| **Ia-A (beta, beta)** | 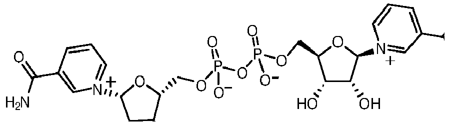 |
| **Ia-B (beta, alpha)** | |
| **Ia-C (alpha, alpha)** | |
| **Ia-D (beta, beta)** | |
| **Ia-E (beta, alpha)** | |
| **Ia-F (alpha, alpha)** | |
or pharmaceutically acceptable salts and solvates thereof or prodrugs thereof.

11. The compound for use according to any one of claims **1** to **10**, said use comprising a step of administering sequentially, simultaneously and/or separately at least another active ingredient selected from an antiviral agent, a neuraminidase inhibitor, a M2 proton channel blocker, an anti-interleukin 6, a JAK inhibitor, an interferon and a mixture thereof, and/or said use comprising a step of administering sequentially, simultaneously and/or separately at least another active ingredient selected from an antiviral agent; an anti-interleukin 6 (anti-IL6) agent; a Janus-associated kinase (JAK) inhibitor; an interferon; a macrolide, preferably selected from the group consisting of azithromycin, clarithromycin, erythromycin, spiramycin, telithromycin, another active ingredient selected from BXT-25, chloroquine, hydroxychloroquine, brilacidin, dehydroandrographolide succinate, APN01, fingolimod, methylprednisolone, thalidomide, bevacizumab, sildenafil citrate, carrimycin and nicotine; and a mixture thereof.

12. Pharmaceutical composition for use in the treatment and/or prevention of viral infections or for use in the treatment and/or prevention of respiratory or extra-respiratory complications and/or infections of viral origin, comprising at least one compound for use according to any one of claims **1** to **11** and at least one pharmaceutically acceptable carrier.

13. Pharmaceutical composition for use according to claim **12**, comprising in addition to at least one compound for use according to any one of claims **1** to **11**, at least one active ingredient selected from an antiviral agent, a neuraminidase inhibitor, a M2 proton channel blocker, an anti-interleukin 6, a JAK inhibitor, an interferon and a mixture thereof, and/or at least another active ingredient selected from an antiviral agent, a neuraminidase inhibitor, a M2 proton channel blocker, an anti-interleukin 6, a JAK inhibitor, an interferon and a mixture thereof, and/or at least another active ingredient selected from an antiviral agent; an anti-interleukin 6 (anti-IL6) agent; a Janus-associated kinase (JAK) inhibitor; an interferon; a macrolide, preferably selected from the group consisting of azithromycin, clarithromycin, erythromycin, spiramycin, telithromycin, another active ingredient selected from BXT-25, chloroquine, hydroxychloroquine, brilacidin, dehydroandrographolide succinate, APN01, fingolimod, methylprednisolone, thalidomide, bevacizumab, sildenafil citrate, carrimycin and nicotine; and a mixture thereof.

14. Pharmaceutical composition, comprising at least one compound according to any one of claims **1, 6, 9** or **10**, and at least one active ingredient selected from an antiviral agent, a neuraminidase inhibitor, a M2 proton channel blocker, an anti-interleukin 6, a JAK inhibitor, an interferon and a mixture thereof, and/or at least another active ingredient selected from an antiviral agent, a neuraminidase inhibitor, a M2 proton channel blocker, an anti-interleukin 6, a JAK inhibitor, an interferon and a mixture thereof, and/or at least another active ingredient selected from an antiviral agent; an anti-interleukin 6 (anti-IL6) agent; a Janus-associated kinase (JAK) inhibitor; an interferon; a macrolide, preferably selected from the group consisting of azithromycin, clarithromycin, erythromycin, spiramycin, telithromycin, another active ingredient selected from BXT-25, chloroquine, hydroxychloroquine, brilacidin, dehydroandrographolide succinate, APN01, fingolimod, methylprednisolone, thalidomide, bevacizumab, sildenafil citrate, carrimycin and nicotine; and a mixture thereof.
